(19) Europäisches Patentamt
European Patent Office
Office européen des brevets

(11) Veröffentlichungsnummer: **0 077 961**
**A2**

# (12) EUROPÄISCHE PATENTANMELDUNG

(21) Anmeldenummer: **82109383.8**

(22) Anmeldetag: **11.10.82**

(51) Int. Cl.³: **C 07 C 147/02**
C 07 C 154/02, C 07 C 149/243
C 07 C 149/18, C 07 C 79/35
C 07 D 231/10, C 07 F 9/40
C 07 F 9/38, C 07 C 149/12
C 07 C 155/02

(30) Priorität: **22.10.81 DE 3141861**

(43) Veröffentlichungstag der Anmeldung:
**04.05.83 Patentblatt 83/18**

(84) Benannte Vertragsstaaten:
**AT BE CH DE FR GB IT LI NL**

(71) Anmelder: **BAYER AG**
**Zentralbereich Patente, Marken und Lizenzen**
**D-5090 Leverkusen 1, Bayerwerk(DE)**

(72) Erfinder: **Förster, Heinz, Dr.**
**Am Eckbusch 47**
**D-5600 Wuppertal 1(DE)**

(72) Erfinder: **Eue, Ludwig, Dr.**
**Paul-Klee-Strasse 36**
**D-5090 Leverkusen 1(DE)**

(72) Erfinder: **Schmidt, Rudolf, Dr.**
**In Waldwinkel 110**
**D-5060 Bergisch-Gladbach 2(DE)**

(72) Erfinder: **Lürssen, Klaus, Dr.**
**August-Kierspel-Strasse 89**
**D-5060 Bergisch-Gladbach 2(DE)**

(54) **Substituierte 2-Nitro-5-(4-trifluormethyl-phenoxy)-toluole, Verfahren zu ihrer Herstellung sowie ihre Verwendung als Herbizide und Pflanzenwuchsregulatoren.**

(57) Substituierte 2-Nitro-5-(4-trifluormethylphenoxy)-toluole der Formel

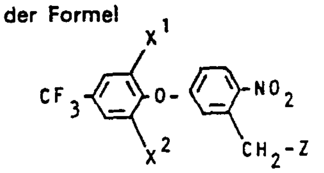

(I)

in welcher

$X^1$, $X^2$ und Z die in der Beschreibung angegebene Bedeutung haben,
mehrere Verfahren zu deren Herstellung und deren Verwendung als Herbizide und Pflanzenwachstumsregulatoren.

2-Nitro-5-(4-trifluormethyl-phenoxy)-benzylthiole der Formel

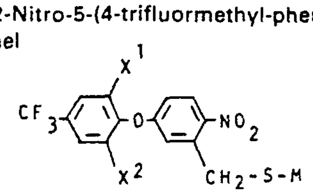

(IV)

in welcher

$X^1$, $X^2$ und M die in der Beschreibung angegebene Bedeutung haben,
ein Verfahren zu deren Herstellung und deren Verwendung als Zwischenprodukte zur Herstellung von Stoffen der Formel (I).

3-(4-Trifluormethyl-phenoxy)-toluole der Formel

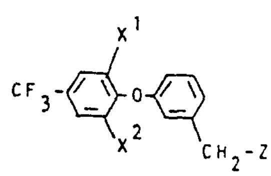

(XI)

in welcher

$X^1$, $X^2$ und Z die in der Beschreibung angegebene Bedeutung haben,
mehrere Verfahren zu deren Herstellung sowie deren Verwendung als Zwischenprodukte zur Synthese von Stoffen der Formel (I).

EP 0 077 961 A2

BAYER AKTIENGESELLSCHAFT 5090 Leverkusen, Bayerwerk
Zentralbereich
Patente, Marken und Lizenzen Dü/bo/c
Ib

Substituierte 2-Nitro-5-(4-trifluormethyl-phenoxy)-toluole, Verfahren zu ihrer Herstellung sowie ihre Verwendung als Herbizide und Pflanzenwuchsregulatoren

Die Erfindung betrifft neue substituierte 2-Nitro-5-(4-trifluormethyl-phenoxy)-toluole, mehrere Verfahren zu deren Herstellung sowie deren Verwendung als Herbizide und Pflanzenwuchsregulatoren.

Es ist bereits bekannt geworden, daß bestimmte substituierte Diphenylether herbizide Eigenschaften besitzen (vgl. US-PS 3 928 416). So läßt sich zum Beispiel 2-Nitro-5-(2,6-dichlor-4-trifluormethyl-phenoxy)-phenol zur Bekämpfung von Unkraut verwenden. Die Wirkung dieses Stoffes ist gut, jedoch ist die Selektivität nicht immer ausreichend.

Es wurden nun neue substituierte 2-Nitro-5-(4-trifluormethyl-phenoxy)-toluole der Formel

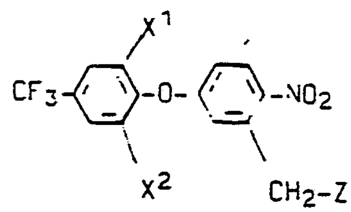

(I)

in welcher

$X^1$ und $X^2$ unabhängig voneinander für Wasserstoff oder Halogen stehen und

Z für Cyano oder für einen gegebenenfalls durch Halogen substituierten Rest aus der Reihe Alkenoxy, Alkinoxy, Benzyloxy, Alkylcarbonyloxy, Alkoxycarbonyloxy, Alkylthio, Alkenylthio, Alkinylthio, Benzylthio, Alkylsulfinyl, Alkenylsulfinyl, Alkinylsulfinyl, Benzylsulfinyl, Alkylsulfonyl, Alkenylsulfonyl, Alkinylsulfonyl, Benzylsulfonyl, Hydroxycarbonylalkylthio, Alkoxycarbonylalkylthio, Alkoxythiocarbonylthio, Dialkylaminocarbonylthio, Alkoxycarbonylalkylsulfinyl, Alkoxycarbonylalkylsulfonyl, Hydroxycarbonylalkylsulfinyl und Hydroxycarbonylalkylsulfonyl, oder für einen gegebenenfalls durch Halogen, Cyano, Nitro, Alkyl, Trifluormethyl, Alkoxy, Alkylthio, Alkylsulfinyl und/oder Alkylsulfonyl substituierten Rest aus der Reihe Phenoxy, Phenylthio, Phenylsulfinyl, Phenylsulfonyl, Pyridinoxy oder Pyridinthio steht,

oder

Z für den Rest der Formel $-S(O)_n-$ 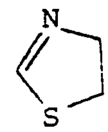 oder

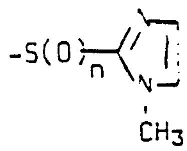 steht, worin

n jeweils für die Zahlen 0, 1 oder 2 steht,

oder

Z für den Rest 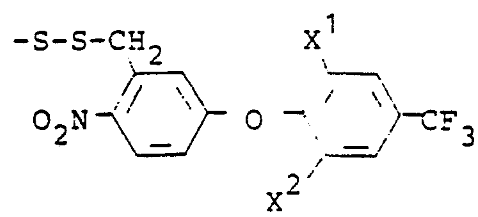

steht, worin

$X^1$ und $X^2$ die oben angegebene Bedeutung haben,

oder

Z für den Rest der Formel 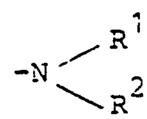 steht, worin

$R^1$ für Wasserstoff, für gegebenenfalls durch Halogen, Cyano oder Alkoxy substituiertes Alkyl, für Alkenyl, Alkinyl, Cycloalkyl oder Benzyl steht und

$R^2$ für Wasserstoff, für gegebenenfalls durch Halogen, Cyano, Alkoxy, Hydroxycarbonyl oder Alkoxycarbonyl substituiertes Alkyl, für Alkenyl, Alkinyl, Cycloalkyl oder

Benzyl steht, oder für Naphthyl oder Phenyl steht, wobei jeder dieser beiden Reste gegebenenfalls durch Halogen, Cyano, Nitro, Alkyl, Trifluormethyl, Alkoxy, Alkylthio, Alkylsulfinyl und/oder Alkylsulfonyl substituiert ist,

oder

$R^1$ und $R^2$ zusammen mit dem Stickstoffatom, an das sie gebunden sind, einen gegebenenfalls durch Halogen, Alkyl, Halogenalkyl, Alkoxy, Alkylthio, Cyano, Alkylcarbonyl, Alkoxycarbonyl und/oder Phenyl substituierten gesättigten oder ungesättigten und gegebenenfalls benzanellierten Mono- oder Bicyclus bilden, der gegebenenfalls Sauerstoff, Schwefel und/oder Stickstoff als weitere Heteroatome enthält,

oder

Z für den Rest der Formel 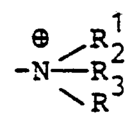 $X^{\ominus}$ steht, worin

$R^1$ und $R^2$ die oben angegebenen Bedeutungen haben,

$R^3$ für Wasserstoff oder Alkyl steht und

X für Halogen steht,

wobei, - für den Fall, daß $R^1$ und $R^2$ zusammen mit dem Stickstoffatom, an das sie gebunden sind, einen Ring bilden, welcher wenigstens ein weiteres Stickstoffatom enthält - , der Rest $R^3$ auch an ein anderes Stickstoffatom gebunden sein kann als an dasjenige, das die Bindung zum übrigen Molekülteil ausübt,

oder

Z auch für den Rest der Formel 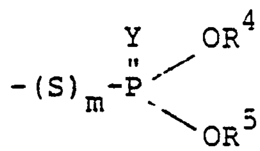 steht, worin

m für die Zahlen 0 oder 1 steht,

Y für Sauerstoff und, - für den Fall, daß m für 1 steht - , auch für Schwefel steht,

$R^4$ für Wasserstoff, Natrium, Kalium oder Alkyl steht und

$R^5$ für Wasserstoff, Natrium, Kalium oder Alalkyl steht, oder zusammen mit $R^4$ für Calcium steht,

gefunden.

Weiterhin wurde gefunden, daß man die neuen substituierten 2-Nitro-5-(4-trifluormethyl-phenoxy)-toluole der Formel (I) erhält, wenn man

(a) 2-Nitro-5-(4-trifluormethyl-phenoxy)-benzylalkohole der Formel

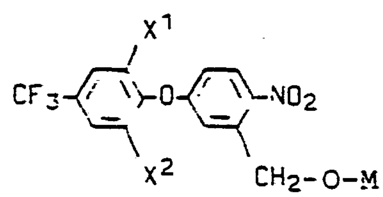 (II)

in welcher

$X^1$ und $X^2$ die oben angegebene Bedeutung haben und

M für Wasserstoff, Natrium oder Kalium steht,

mit Halogenverbindungen der Formel

$$Hal-Z^1 \qquad (III)$$

in welcher

Hal für Chlor, Brom oder Iod steht und

$Z^1$ für einen gegebenenfalls durch Halogen substituierten Rest aus der Reihe Alkenyl, Alkinyl, Benzyl, Alkylcarbonyl oder Alkoxycarbonyl steht,

gegebenenfalls in Gegenwart eines Säureakzeptors und gegebenenfalls in Gegenwart eines Verdünnungsmittels umsetzt,

oder

(b) 2-Nitro-5-(4-trifluormethyl-phenoxy)-benzyl-thiole der Formel

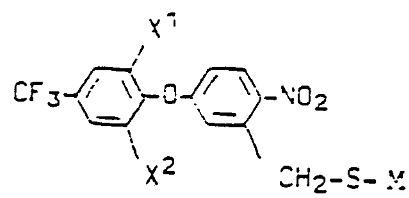

(IV)

in welcher

$X^1$, $X^2$ und M die oben angegebene Bedeutung haben,

mit Halogenverbindungen der Formel

$$Hal-Z^2 \qquad (V)$$

in welcher

Hal die oben angegebene Bedeutung hat und

$Z^2$ für einen gegebenenfalls durch Halogen substituierten Rest aus der Reihe Alkenyl, Alkinyl, Benzyl, Alkyl oder Dialkylaminocarbonyl steht,

gegebenenfalls in Gegenwart eines Säureakzeptors und gegebenenfalls in Gegenwart eines Verdünnungsmittels umsetzt,

oder

(c) 2-Nitro-5-(4-trifluormethyl-phenoxy)-benzylthioverbindungen der Formel

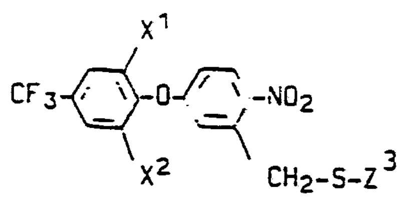

(Ia)

in welcher

$X^1$ und $X^2$ die oben angegebene Bedeutung haben und

$Z^3$ für Wasserstoff oder einen gegebenenfalls durch Halogen substituierten Rest aus der Reihe Alkyl, Alkenyl, Alkinyl, Benzyl, Hydroxycarbonylalkyl oder Alkoxycarbonylalkyl steht, oder für gegebenenfalls durch Halogen, Cyano, Nitro, Alkyl, Trifluormethyl, Alkoxy, Alkylthio, Alkylsulfinyl und/oder Alkylsulfonyl substituiertes Phenyl steht oder für den Rest der Formel

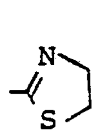 oder 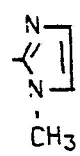 steht,

mit Oxidationsmitteln gegebenenfalls in Gegenwart eines Verdünnungsmittels umsetzt,

oder

(d) 2-Nitro-5-(4-trifluormethyl-phenoxy)-benzylhalogenide der Formel

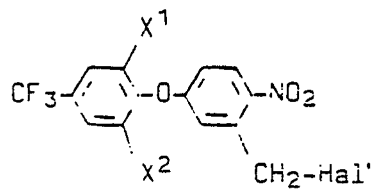

(VI)

in welcher

$X^1$ und $X^2$ die oben angegebene Bedeutung haben und

Hal' für Chlor, Brom oder Iod steht,

α) mit Nucleophilen der Formel

$$M^1\text{-}Z \qquad \text{(VII)}$$

in welcher

Z die oben angegebene Bedeutung hat und

$M^1$ für Wasserstoff, Natrium, Kalium oder Ammonium steht,

gegebenenfalls in Gegenwart eines Säureakzeptors sowie gegebenenfalls in Gegenwart eines Verdünnungsmittels umsetzt,

oder

ß) mit Trialkylphosphiten der Formel

$$P(OR)_3 \qquad (VIII)$$

in welcher

R für Alkyl steht,

gegebenenfalls in Gegenwart eines Katalysators und gegebenenfalls in Gegenwart eines Verdünnungsmittels umsetzt und gegebenenfalls anschließend die dabei entstehenden Phosphonsäureester der Formel

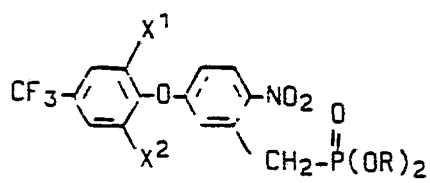

(Ib)

in welcher

$X^1$, $X^2$ und R die oben angegebene Bedeutung haben,

gegebenenfalls in Gegenwart eines Verdünnungsmittels verseift und gegebenenfalls die dabei entstehenden Säuren der Formel

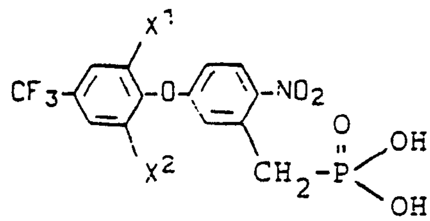

(Ic)

in welcher

$X^1$ und $X^2$ die oben angegebene Bedeutung haben,

mit einer Metallverbindung der Formel

$$M^2-X^3 \quad (IX)$$

in welcher

$M^2$ für Natrium, Kalium oder ein Calcium-äquivalent steht und

$X^3$ für Chlor oder Hydroxy steht,

gegebenenfalls in Gegenwart eines Verdünnungsmittels umsetzt,

oder

(e) 2-Nitro-5-(4-trifluormethyl-phenoxy)-benzylamine der Formel

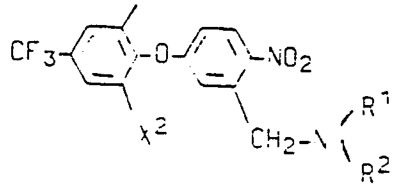

(Id)

in welcher

$X^1$, $X^2$, $R^1$ und $R^2$ die oben angegebene Bedeutung haben,

mit Halogeniden der Formel

Hal"-$R^3$ (X)

in welcher

$R^3$ die oben angegebene Bedeutung hat und

Hal" für Chlor, Brom oder Iod steht,

gegebenenfalls in Gegenwart eines Verdünnungsmittels umsetzt,

oder

(f) substituierte 2-Nitro-5-(4-trifluormethyl-phenoxy)-toluole der Formel

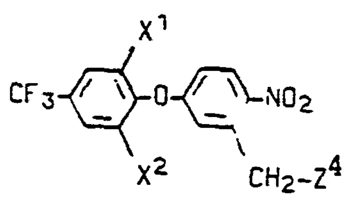

(Ie)

in welcher

$X^1$ und $X^2$ die oben angegebene Bedeutung haben und

$Z^4$ für Alkoxycarbonylalkylthio oder Alkoxycarbonylalkylamino steht,

mit Alkalihydroxiden in Gegenwart eines Verdünnungsmittels umsetzt und die dabei entstehenden Carbonsäuresalze mit Mineralsäuren behandelt,

oder

(g) 3-(4-Trifluormethyl-phenoxy)-toluole der Formel

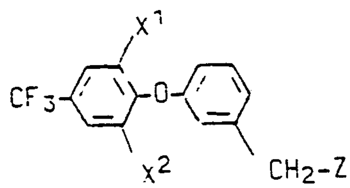

(XI)

in welcher

$X^1$, $X^2$ und Z die oben angegebene Bedeutung haben,

mit Salpetersäure gegebenenfalls in Gegenwart eines Katalysators und gegebenenfalls in Gegenwart eines Verdünnungsmittels umsetzt.

Schließlich wurde gefunden, daß sich die neuen substituierten 2-Nitro-5-(4-trifluormethyl-phenoxy)-toluole der Formel (I) durch hervorragende herbizide und pflanzenwuchsregulierende Eigenschaften auszeichnen.

Überraschenderweise besitzen die erfindungsgemäßen substituierten 2-Nitro-5-(4-trifluormethyl-phenoxy)-toluole der Formel (I) eine wesentlich bessere selektive herbizide Wirksamkeit als das 2-Nitro-5-(2,6-dichlor-4-trifluormethyl-phenoxy)-phenol, welches ein konstitutionell ähnlicher, vorbekannter Wirkstoff gleicher Wirkungsrichtung ist. Darüber hinaus zeichnen sich die erfindungsgemäßen Stoffe unerwarteterweise auch durch eine sehr gute pflanzenwachstumsregulierende Wirksamkeit aus.

Die erfindungsgemäßen substituierten 2-Nitro-5-(4-trifluormethyl-phenoxy)-toluole sind durch die Formel (I) allgemein definiert.

In dieser Formel stehen vorzugsweise

$X^1$ für Chlor,

$X^2$ für Wasserstoff oder Chlor und

Z für Cyano oder für einen gegebenenfalls durch Fluor und/oder Chlor substituierten Rest aus der Reihe $C_3$-$C_5$-Alkenoxy, $C_3$-$C_5$-Alkinoxy, Benzyloxy, $C_1$-$C_4$-Alkyl-carbonyloxy, $C_1$-$C_4$-Alkoxycarbonyloxy, $C_1$-$C_4$-Alkylthio, $C_3$-$C_5$-Alkenylthio, $C_3$-$C_5$-Alkinylthio, Benzylthio, $C_1$-$C_4$-Alkylsulfinyl, $C_3$-$C_5$-Alkenylsulfinyl, $C_3$-$C_5$-Alkinylsulfinyl, Benzylsulfinyl, $C_1$-$C_4$-Alkylsulfonyl, $C_3$-$C_5$-Alkenylsulfonyl, $C_3$-$C_5$-Alkinylsulfonyl, Benzylsulfonyl, Hydroxycarbonyl-$C_1$-$C_2$-alkylthio, $C_1$-$C_4$-Alkoxycarbonyl-$C_1$-$C_2$-alkyl-

thio, $C_1$-$C_4$-Alkoxy-thiocarbonylthio oder Di-($C_1$-$C_4$-alkyl)-aminocarbonylthio, oder für einen gegebenenfalls durch Fluor, Chlor, Brom, Cyano, Nitro, $C_1$-$C_4$-Alkyl, Trifluormethyl, $C_1$-$C_4$-Alkoxy, $C_1$-$C_4$-Alkylthio, $C_1$-$C_4$-Alkylsulfinyl und/oder $C_1$-$C_4$-Alkylsulfonyl substituierten Rest aus der Reihe Phenoxy, Phenylthio, Phenylsulfinyl, Phenylsulfonyl, Pyridinoxy oder Pyridinthio,

oder

Z steht für den Rest der Formel 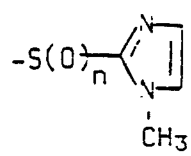

oder 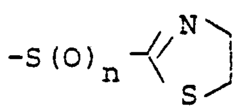

worin

n jeweils für die Zahlen 0, 1 oder 2 steht,

oder

Z steht für den Rest der Formel

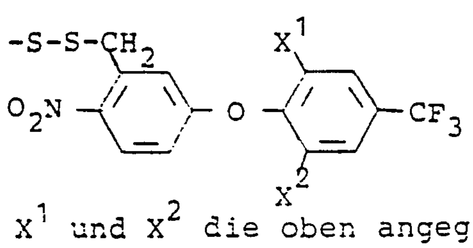

, worin

$X^1$ und $X^2$ die oben angegebene Bedeutung haben,

oder

Z steht für den Rest der Formel $-N\begin{smallmatrix}R^1\\R^2\end{smallmatrix}$ , worin

$R^1$ für Wasserstoff, für gegebenenfalls durch Fluor, Chlor, Cyano oder $C_1$-$C_4$-Alkoxy substituiertes $C_1$-$C_4$-Alkyl, für $C_3$-$C_5$-Alkenyl, $C_3$-$C_5$-Alkinyl, $C_5$-$C_6$-Cycloalkyl oder Benzyl steht und

$R^2$ für Wasserstoff, für gegebenenfalls durch Fluor, Chlor, Cyano, $C_1$-$C_4$-Alkoxy, Hydroxycarbonyl oder $C_1$-$C_4$-Alkoxy-carbonyl substituiertes $C_1$-$C_4$-Alkyl, für $C_3$-$C_5$-Alkenyl, $C_3$-$C_5$-Alkinyl, $C_5$-$C_6$-Cycloalkyl oder Benzyl steht, oder für Naphthyl oder Phenyl steht, wobei jeder der beiden letztgenannten Reste gegebenenfalls durch Fluor, Chlor, Brom, Cyano, Nitro, $C_1$-$C_4$-Alkyl, Trifluormethyl, $C_1$-$C_4$-Alkoxy, $C_1$-$C_4$-Alkylthio, $C_1$-$C_4$-Alkylsulfinyl und/oder $C_1$-$C_4$-Alkylsulfonyl substituiert ist,

oder

$R^1$ und $R^2$ zusammen mit dem Stickstoffatom, an das sie gebunden sind, einen gegebenenfalls durch Fluor, Chlor, Brom, Iod, Cyano, $C_1$-$C_4$-Alkyl, Trifluormethyl, $C_1$-$C_4$-Alkoxy, $C_1$-$C_4$-Alkylthio, $C_1$-$C_4$-Alkyl-carbonyl, $C_1$-$C_4$-Alkoxy-carbonyl und/oder Phenyl substituierten gesättigten oder ungesättigten und gegebenenfalls benzannellierten Mono- oder Bicyclus bilden, welcher 3 bis 12 Kohlenstoffatome und gegebenenfalls Sauerstoff, Schwefel und/oder Stickstoff als weitere Heteroatome enthält,

oder

Z für den Rest der Formel 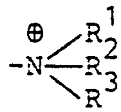 $X^{\ominus}$, worin

$R^1$ und $R^2$ die oben angegebenen Bedeutungen haben,

$R^3$ für Wasserstoff oder $C_1$-$C_4$-Alkyl steht und

X für Chlor, Brom oder Iod steht,

wobei, - für den Fall, daß $R^1$ und $R^2$ zusammen mit dem Stickstoffatom, an das sie gebunden sind, einen Ring bilden, welcher wenigstens ein weiteres Stickstoffatom enthält - , der Rest $R^3$ auch an ein anderes Stickstoffatom gebunden sein kann als an dasjenige, das die Bindung zum übrigen Molekülteil ausübt,

oder

Z steht auch für den Rest der Formel 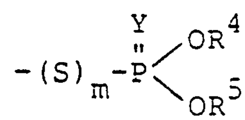 , worin

m für die Zahlen 0 oder 1 steht,

Y für Sauerstoff und - für den Fall, daß m für 1 steht - , auch für Schwefel steht,

$R^4$ für Wasserstoff, Natrium, Kalium oder $C_1$-$C_4$-Alkyl steht und

$R^5$ für Wasserstoff, Natrium, Kalium oder $C_1$-$C_4$-Alkyl steht oder zusammen mit $R^4$ für Calcium steht.

Eine besonders bevorzugte Gruppe von Verbindungen der Formel (I) sind diejenigen Stoffe, in denen

$X^1$ für Chlor steht,

$X^2$ für Wasserstoff oder Chlor steht und

Z für Cyano oder für gegebenenfalls durch Fluor und/oder Chlor substituiertes Allyloxy, Propargyloxy, Benzyloxy, Acetoxy, Methoxycarbonyloxy oder Ethoxycarbonyloxy steht.

Eine weitere Gruppe besonders bevorzugter Verbindungen der Formel (I) sind diejenigen Stoffe, in denen

$X^1$ für Chlor steht,

$X^2$ für Wasserstoff oder Chlor steht und

Z für gegebenenfalls durch Fluor und/oder Chlor substituiertes Methylthio, Ethylthio, Propylthio, Butylthio, Allylthio, Propargylthio, Benzylthio, Methylsulfinyl, Ethylsulfinyl, Propylsulfinyl, Butylsulfinyl, Allylsulfinyl, Propargylsulfinyl, Benzylsulfinyl, Methylsulfonyl, Ethylsulfonyl, Propylsulfonyl, Butylsulfonyl, Allylsulfonyl,

Propargylsulfonyl, Benzylsulfonyl, Hydroxycarbonyl-methylthio, Methoxycarbonylmethylthio, Ethoxycarbonylmethylthio, Hydroxycarbonylethylthio, Methoxycarbonylethylthio, Methoxythiocarbonylthio, Ethoxythiocarbonylthio, Dimethylaminocarbonylthio steht oder für den Rest der Formel

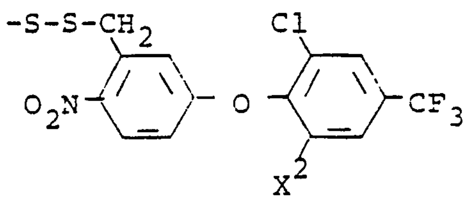

steht,

worin $X^2$ für Wasserstoff oder Chlor steht.

Eine andere Gruppe besonders bevorzugter Verbindungen sind diejenigen Stoffe der Formel (I), in denen

$X^1$ für Chlor steht,

$X^2$ für Wasserstoff oder Chlor steht und

Z für einen gegebenenfalls durch Fluor, Chlor, Brom, Cyano, Nitro, Methyl, Trifluormethyl, Methoxy, Methylthio, Methylsulfinyl und/oder Methylsulfonyl substituierten Rest aus der Reihe Phenoxy, Phenylthio, Phenylsulfinyl, Phenylsulfonyl, Pyridinoxy oder Pyridinthio steht

oder

Z für den Rest der Formel $-S(O)_n$ 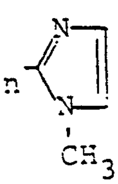 oder $-S(O)_n$ 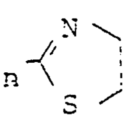 steht, worin

n jeweils für die Zahlen 0, 1 oder 2 steht.

Eine besonders bevorzugte Gruppe von Verbindungen der Formel (I) sind außerdem diejenigen Stoffe, in denen

$X^1$ für Chlor steht,

$X^2$ für Wasserstoff oder Chlor steht und

Z für den Rest der Formel 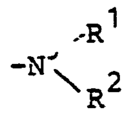 steht, worin

$R^1$ für Wasserstoff, Methyl, Ethyl, Propyl, Butyl, 2-Chlorethyl, 2-Cyanoethyl, 2-Methoxyethyl, Allyl, Propargyl, Cyclopentyl oder Benzyl steht und

$R^2$ für Wasserstoff, Methyl, Ethyl, Propyl, Butyl, 2-Chlorethyl, 2-Cyanoethyl, 2-Methoxyethyl, Hydroxycarbonylmethyl, Methoxycarbonylmethyl, Ethoxycarbonylmethyl, Allyl, Propargyl, Cyclopentyl, Cyclohexyl, Benzyl, 1-Methylpropargyl, 1,1-Dimethyl-propargyl oder Phenyl steht, welches gegebenenfalls durch Fluor, Chlor, Brom, Cyano, Nitro, Methyl, Trifluormethyl, Methoxy, Ethoxy, Methylthio, Methylsulfinyl oder Methylsulfonyl substituiert ist,

oder

$R^1$ und $R^2$ zusammen mit dem Stickstoffatom, an das sie gebunden sind, für gegebenenfalls durch Methyl und/oder Ethyl substituierte Reste aus der Reihe Pyrrolidyl, Piperidyl, Morpholinyl, Perhydroazepinyl, Indolinyl, Perhydroindolyl,

Tetrahydrochinolyl, Tetrahydroisochinolyl, Perhydrochinolyl und Perhydroisochinolyl, oder für gegebenenfalls durch Chlor, Brom, Methyl, Ethyl, Trifluormethyl, Chlormethyl, Methoxy, Methylthio, Cyano, Acetyl und/oder Phenyl substituiertes Pyrazolyl, Imidazolyl oder Triazolyl stehen.

Eine besonders bevorzugte Gruppe von Verbindungen der Formel (I) sind außerdem diejenigen Stoffe, in denen

$X^1$ für Chlor,

$X^2$ für Wasserstoff oder Chlor und

Z für den Rest der Formel 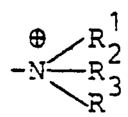 $Cl^{\ominus}$ steht, worin

$R^1$ für Wasserstoff, Methyl, Ethyl, Propyl, Butyl, 2-Chlorethyl, 2-Cyanoethyl, 2-Methoxyethyl, Allyl, Propargyl, Cyclopentyl oder Benzyl steht und

$R^2$ für Wasserstoff, Methyl, Ethyl, Propyl, Butyl, 2-Chlorethyl, 2-Cyanoethyl, 2-Methoxyethyl, Hydroxycarbonylmethyl, Methoxycarbonylmethyl, Ethoxycarbonylmethyl, Allyl, Propargyl, Cyclopentyl, Cyclohexyl, Benzyl, 1-Methylpropargyl, 1,1-Dimethyl-propargyl oder Phenyl steht, welches gegebenenfalls durch Fluor, Chlor, Brom, Cyano, Nitro, Methyl, Trifluormethyl, Methoxy, Ethoxy, Methylthio, Methylsulfinyl, oder Methylsulfonyl substituiert ist,

oder

$R^1$ und $R^2$ zusammen mit dem Stickstoffatom, an das sie gebunden sind, für gegebenenfalls durch Methyl und/oder Ethyl substituierte Reste aus der Reihe Pyrrolidyl, Piperidyl, Morpholinyl, Perhydroazepinyl, Indolinyl, Perhydroindolyl, Tetrahydrochinolyl, Tetrahydroisochinolyl, Perhydrochinolyl und Perhydroisochinolyl, oder für gegebenenfalls durch Chlor, Brom, Methyl, Ethyl, Trifluormethyl, Chlormethyl, Methoxy, Methylthio, Cyano, Acetyl und/oder Phenyl substituiertes Pyrazolyl, Imidazolyl oder Triazolyl stehen,

$R^3$ für Wasserstoff, Methyl oder Ethyl steht und

X für Chlor, Brom oder Iod steht.

Eine besonders bevorzugte Gruppe von Verbindungen der Formel (I) sind schließlich auch diejenigen Stoffe, in denen

$X^1$ für Chlor steht,

$X^2$ für Wasserstoff oder Chlor steht und

Z für den Rest der Formel 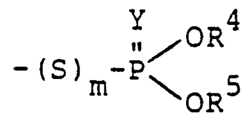 steht, worin

m für die Zahlen 0 oder 1 steht,

Y für Sauerstoff und, - für den Fall, daß m für 1 steht - , auch für Schwefel steht,

$R^4$ für Wasserstoff, Natrium, Kalium, Methyl oder Ethyl steht und

$R^5$ für Wasserstoff, Methyl oder Ethyl steht oder

$R^4$ und $R^5$ gemeinsam für Calcium stehen.

Als Beispiele für Verbindungen der Formel (I) seien im einzelnen diejenigen Stoffe genannt, die in der nachfolgenden Tabelle formelmäßig aufgeführt sind.

Tabelle 1

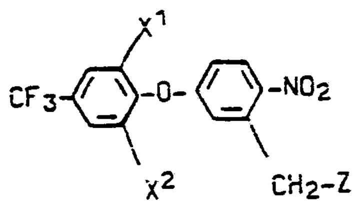

(I)

| $X^2$ | $X^1$ | Z |
|---|---|---|
| H | Cl | $-O-CH_2-CH=CH_2$ |
| H | Cl | $-O-CH_2-C{\equiv}CH$ |
| Cl | Cl | $-O-CH_2-C{\equiv}CH$ |
| Cl | Cl | $-SCH_3$ |
| H | Cl | $-SCH_3$ |
| H | Cl | $-SC_2H_5$ |
| H | Cl | $-SC_3H_7-n$ |
| H | Cl | $-SC_3H_7-iso$ |
| Cl | Cl | $-SC_3H_7-iso$ |
| Cl | Cl | $-S-CH_2-CO-OH$ |

Tabelle 1 (Fortsetzung)

| $X^2$ | $X^1$ | Z |
|---|---|---|
| Cl | Cl | $-S-CH_2-CO-OCH_3$ |
| H | Cl | $-S(O)-C_2H_5$ |
| Cl | Cl | $-S(O)_2-C_2H_5$ |
| Cl | Cl | $-O-CO-CH_3$ |
| H | Cl | 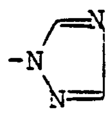 |
| H | Cl | $-NH_2$ |
| H | Cl | $-\overset{\oplus}{N}(C_2H_5)_3Cl^{\ominus}$ |
| H | Cl | 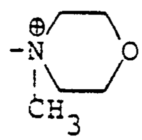 $Cl^{\ominus}$ |
| H | Cl | $-\overset{\oplus}{\underset{CH_3}{N}}(CH_2-CH_2-Cl)_2 \quad Cl^{\ominus}$ |
| H | Cl | 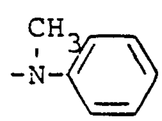 |
| H | Cl | $-NH-CH_2-CO-OCH_3$ |

Verwendet man 2-Nitro-5-(2,6-dichlor-4-trifluormethyl-phenoxy)-benzylalkohol und Acetylchlorid als Ausgangsstoffe, so kann der Verlauf des erfindungsgemäßen Verfahrens (a) durch das folgende Formelschema wiedergegeben werden:

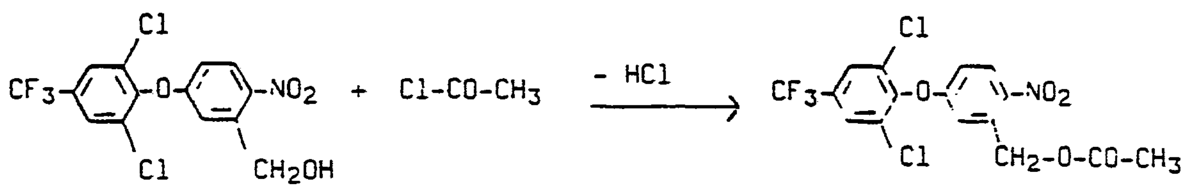


Verwendet man Natrium-[2-Nitro-5-(2,6-dichlor-4-trifluormethyl-phenoxy)-benzylthiolat] und Benzylchlorid als Ausgangsstoffe, so kann der Verlauf des erfindungsgemäßen Verfahrens (b) durch das folgende Formelschema wiedergegeben werden:

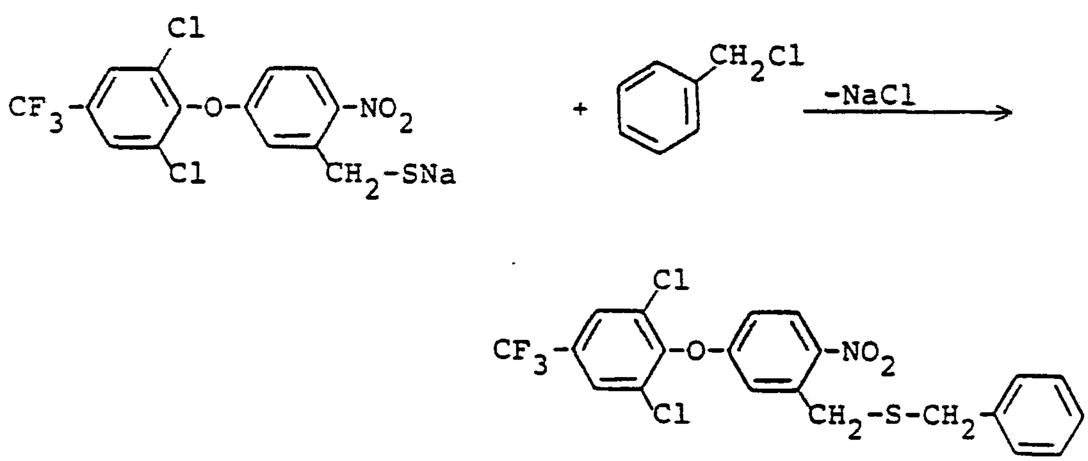


Verwendet man (2-Nitro-5-(2,6-dichlor-4-trifluormethyl-phenoxy)-benzyl)-methylsulfid als Ausgangsstoff und Wasserstoffperoxid als Oxidationsmittel, so kann der Verlauf des erfindungsgemäßen Verfahrens (c) durch das folgende Formelschema wiedergegeben werden:

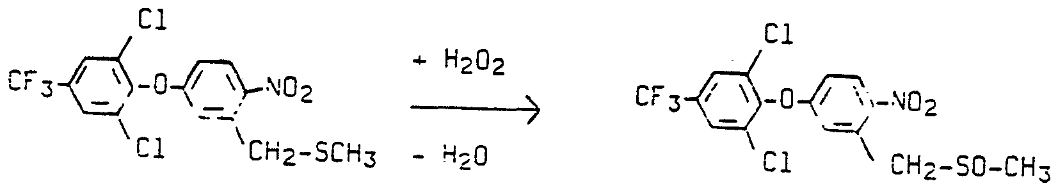


Verwendet man 2-Nitro-5-(2-chlor-4-trifluormethyl-phenoxy)-benzylbromid und Dimethylamin als Ausgangsstoffe, so kann der Verlauf des erfindungsgemäßen Verfahrens (d), Variante $\alpha$, durch das folgende Formelschema wiedergegeben werden:

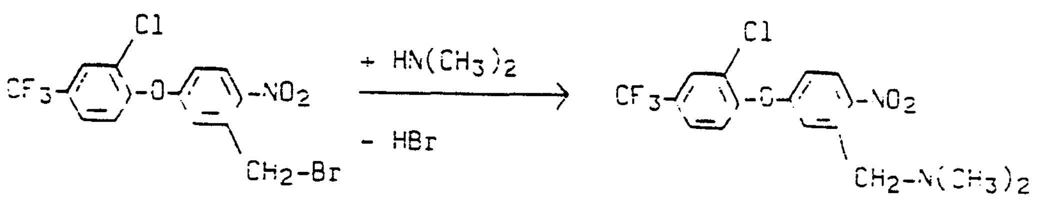


Verwendet man 2-Nitro-5-(2-chlor-4-trifluormethyl-phenoxy)-benzylchlorid und Trimethylphosphit als Ausgangsstoffe, so kann der Verlauf des erfindungsgemäßen Verfahrens (d), Variante ß, durch das folgende Formelschema wiedergegeben werden:

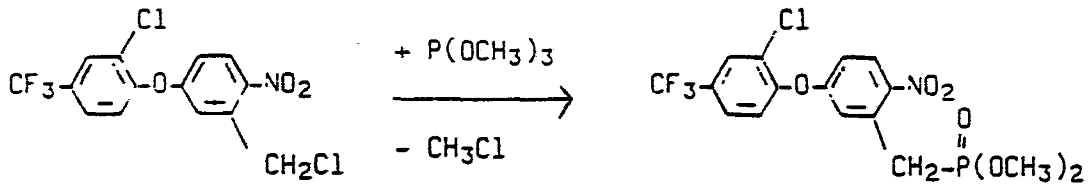


Verwendet man N-(2-Nitro-5-(2,6-dichlor-4-trifluormethyl-phenoxy)-benzyl)-piperidin und Ethylbromid als Ausgangsstoffe, so kann der Verlauf des erfindungsgemäßen Verfahrens (e) durch das folgende Formelschema wiedergegeben werden:

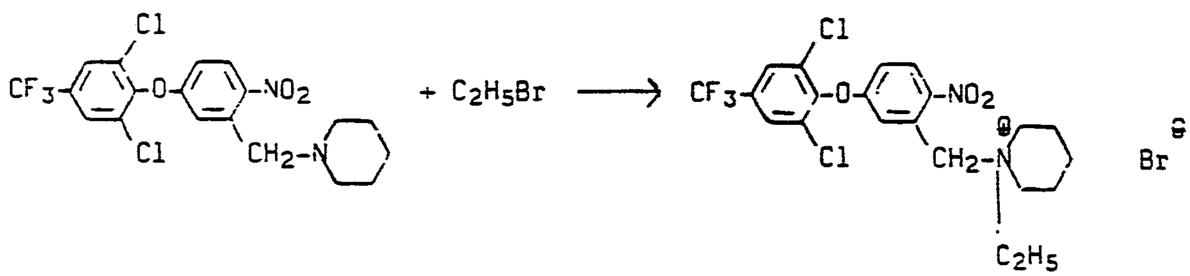


Verwendet man α-(Methoxycarbonyl-methylthio)-2-nitro-5-(2,6-dichlor-4-trifluormethyl-phenoxy)-toluol als Ausgangsstoff und Natronlauge und Salzsäure als Reaktionskomponenten, so kann der Verlauf des erfindungsgemäßen Verfahrens (f) durch das folgende Reaktionsschema wiedergegeben werden:

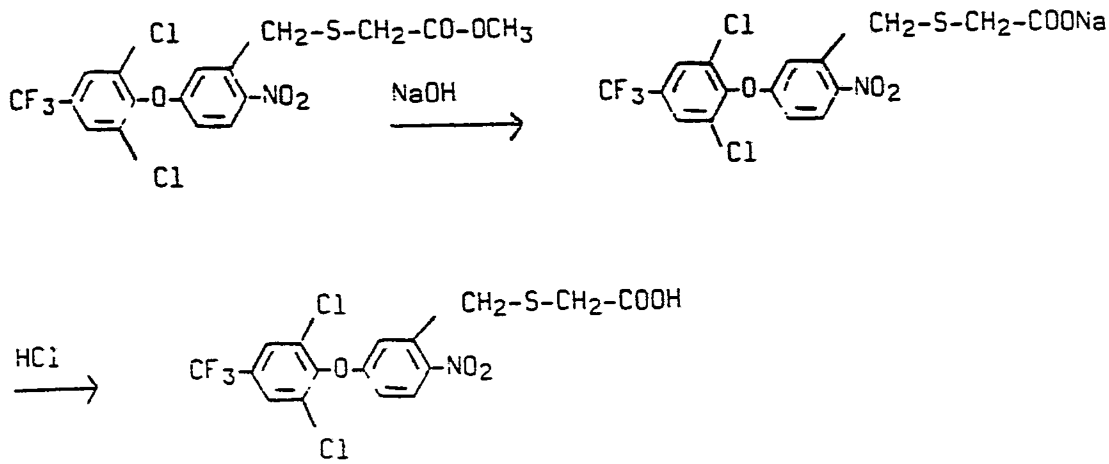

Verwendet man ∕ -(Methoxycarbonyl-methyl-sulfonyl)-3-(2-chlor-4-trifluormethyl-phenoxy)-toluol als Ausgangsstoff und Salpetersäure als Nitrierungsmittel, so kann der Verlauf des erfindungsgemäßen Verfahrens (g) durch das folgende Formelschema wiedergegeben werden:

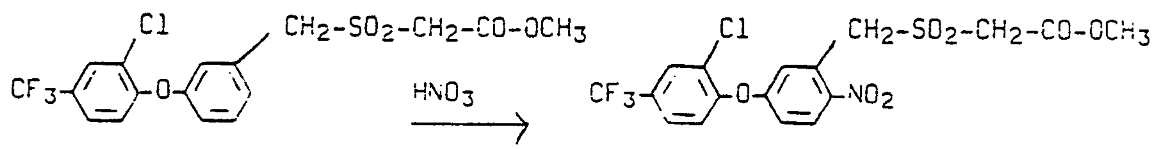


Die bei der Durchführung des erfindungsgemäßen Verfahrens (a) als Ausgangsstoffe benötigten 2-Nitro-5-(4-trifluormethyl-phenoxy)-benzylalkohole sind durch die Formel (II) definiert.

In dieser Formel stehen vorzugsweise

$X^1$ für Chlor,

$X^2$ für Wasserstoff oder Chlor und

M für Wasserstoff, Natrium oder Kalium.

Als Beispiele für die Verbindungen der Formel (II) seien genannt: 2-Nitro-5-(2-chlor-4-trifluormethyl-phenoxy)-benzylalkohol, 2-Nitro-5-(2,6-dichlor-4-trifluormethyl-phenoxy)-benzylalkohol sowie die entsprechenden Natrium- und Kaliumsalze.

Die Verbindungen der Formel (II) sind bisher noch nicht bekannt. Sie lassen sich herstellen, indem man 2-Nitro-5-(4-trifluormethyl-phenoxy)-benzoesäurechloride bzw. entsprechende Aldehyde der Formel

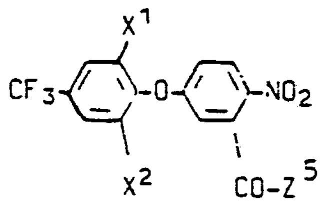

(XII)

in welcher

$X^1$ und $X^2$ die oben angegebenen Bedeutungen haben und

$Z^5$ für Wasserstoff oder Chlor steht,

mit Hydridkomplexen, wie z.B. Natriumboranat, in Gegenwart von Verdünnungsmitteln, wie z.B. Diglycoldimethylether (Diglyme), bei Temperaturen zwischen -20 und +20°C umsetzt. Die Aufarbeitung kann wie üblich durchgeführt werden, beispielsweise durch Verdünnen mit Wasser, Ansäuern, z.B. mit Essigsäure, und Isolieren der kristallin anfallenden Produkte der Formel (II) durch Filtration.

Die Verbindungen der Formel (XII) sind bereits bekannt (vergleiche DE-OS 2 950 401 und 3 017 795).

Die bei dem erfindungsgemäßen Verfahren (a) weiterhin als Ausgangsstoffe zu verwendenden Halogenverbindungen sind durch die Formel (III) definiert. In dieser Formel steht Hal für Chlor, Brom oder Iod. $Z^1$ steht vorzugsweise für gegebenenfalls durch Chlor substituiertes $C_3$-$C_5$-Alkenyl, $C_3$-$C_5$-Alkinyl, Benzyl, $C_1$-$C_4$-Alkylcarbonyl oder $C_1$-$C_4$-Alkoxy-carbonyl.

Als Beispiele für Verbindungen der Formel (III) seien genannt: Allylbromid, Propargylbromid, Benzylchlorid, Acetylchlorid, Propionylchlorid, Chlorameisensäuremethylester und Chlorameisensäureethylester.

Die Verbindungen der Formel (III) sind bekannt.

Das erfindungsgemäße Verfahren (a) wird vorzugsweise unter Verwendung von Verdünnungsmitteln durchgeführt.

Als Verdünnungsmittel kommen praktisch alle inerten organischen Lösungsmittel in Frage. Hierzu gehören insbesondere aliphatische und aromatische, gegebenenfalls halogenierte Kohlenwasserstoffe, wie Pentan, Hexan, Heptan, Cyclohexan, Petrolether, Benzin, Ligroin, Benzol, Toluol, Xylol, Methylenchlorid, Ethylenchlorid, Chloroform, Tetrachlorkohlenstoff, Chlorbenzol und o-Dichlorbenzol, Ether wie Diethyl- und Dibutylether, Glykoldimethylether, Diglykoldimethylether, Tetrahydrofuran und Dioxan, Ketone, wie Aceton, Methylethyl-, Methyl-isopropyl- und Methyl-isobutylketon, Ester, wie Essigsäure-methylester und -ethylester, Nitrile, wie z.B. Acetonitril und Propionitril, Amide, wie z.B. Dimethylformamid, Dimethylacetamid und N-Methyl-pyrrolidon, sowie Dimethylsulfoxid, Tetramethylensulfon und Hexamethylphosphorsäuretriamid.

Bei der Durchführung des erfindungsgemäßen Verfahrens (a) arbeitet man gegebenenfalls in Gegenwart eines Säureakzeptors. Als solche können die üblichen Säurebindemittel verwendet werden. Vorzugsweise in Frage kommen Alkalihydroxide, wie z.B. Natriumhydroxid und Kaliumhydroxid, Erdalkalihydroxide, wie z.B. Calciumhydroxid, Alkalicarbonate, wie z.B. Natriumcarbonat und Kaliumcarbonat, sowie aliphatische, aromatische und heterocyclische Amine, wie z.B. Triethylamin, Dimethylamin, Dimethylbenzylamin, Diazabicyclononen (DBN), Diazabicycloundecen (DBU) und Pyridin.

Die Reaktionstemperaturen können bei der Durchführung des erfindungsgemäßen Verfahrens (a) innerhalb eines größeren Bereichs variiert werden. Im allgemeinen arbeitet man bei Temperaturen zwischen 0 und 150°C, vorzugsweise zwischen 10 und 100°C.

Das erfindungsgemäße Verfahren (a) wird im allgemeinen bei Normaldruck oder bei erhöhtem Druck bis etwa 100 bar durchgeführt.

Zur Durchführung des erfindungsgemäßen Verfahrens (a) setzt man auf 1 Mol 2-Nitro-5-(4-trifluormethyl-phenoxy)-benzylalkohol der Formel (II) im allgemeinen 1 bis 3 Mol Halogenverbindung der Formel (III) ein. Die Reaktionskomponenten werden bei Raumtemperatur gegebenenfalls unter Zugabe eines Säureakzeptors und eines Verdünnungsmittels vermischt und gegebenenfalls bei erhöhter Temperatur bis zum Ende der Umsetzung gerührt.

Die Aufarbeitung erfolgt nach üblichen Methoden. Bei einer bevorzugten Aufarbeitungsweise wird das Reaktionsgemisch mit Wasser verdünnt. Soweit hierbei das Produkt der Formel (I) kristallin anfällt, wird es durch Absaugen isoliert. Fällt das Produkt in öliger Form an, wird es mit einem mit Wasser praktisch nicht mischbaren organischen Lösungsmittel, wie z.B. Cyclohexan extrahiert. Die Extraktionslösung wird mit Wasser neutral gewaschen, getrocknet, filtriert und eingeengt. Das nach sorgfältigem Abziehen der flüchtigen Bestandteile zurückbleibende Produkt wird durch seinen Brechungsindex charakterisiert.

Die bei dem erfindungsgemäßen Verfahren (b) als Ausgangsstoffe benötigten 2-Nitro-5-(4-trifluormethyl-phenoxy)-benzyl-thiole sind durch die Formel (IV) definiert. In dieser Formel stehen vorzugsweise $X^1$ für Chlor, $X^2$ für Wasserstoff oder Chlor und M für Wasserstoff, Natrium oder Kalium.

Als Beispiele für Verbindungen der Formel (IV) seien genannt: 2-Nitro-5-(2-chlor-4-trifluormethyl-phenoxy)-benzylmercaptan und 2-Nitro-5-(2,6-dichlor-4-trifluormethyl-phenoxy)-benzylmercaptan sowie die entsprechenden Natrium- und Kalium-Salze.

Die Verbindungen der Formel (IV) sind bisher noch nicht bekannt. Sie lassen sich jedoch herstellen, indem man Bis-(2-Nitro-5-(4-trifluormethyl-phenoxy)-benzyl)-disulfide der Formel

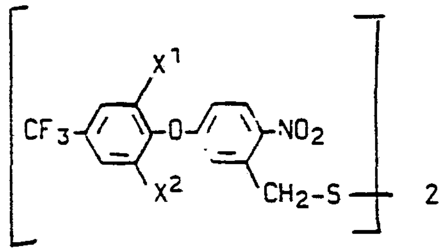

(I f)

in welcher

$X^1$ und $X^2$ die oben angegebene Bedeutung haben,

mit Natriumsulfid und/oder Natriumdisulfid und Natriumhydroxid in Gegenwart von Wasser und gegebenenfalls in Gegenwart eines organischen Lösungsmittels, wie z.B. Ethanol, bei Temperaturen zwischen 2o und loo°C umsetzt. Die Aufarbeitung erfolgt nach üblichen Methoden, beispielsweise durch Eingießen des Reaktionsgemisches in Eiswasser, Ansäuern mit Salzsäure, Extrahieren des Produktes mit einem mit Wasser praktisch nicht mischbaren Lösungsmittel, wie z.B. Toluol, und Abdestillieren des Extraktionsmittels unter vermindertem Druck.

Die Verbindungen der Formel (I f) sind bisher noch nicht bekannt. Sie lassen sich herstellen, indem man S-(2-Nitro-5-(4-trifluormethyl-phenoxy)-benzyl)-O-alkyl-dithiokohlensäureester der Formel

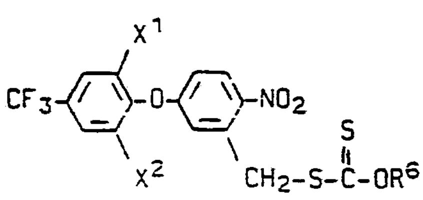

(I g)

in welcher

$X^1$ und $X^2$ die oben angegebene Bedeutung haben und

$R^6$ für Methyl oder Ethyl steht,

mit wässrigem Ammoniak gegebenenfalls in Gegenwart eines organischen Lösungsmittels, wie z.B. Ethanol, unter Einwirkung von Luftsauerstoff bei Temperaturen zwischen 1o und 5o°C umsetzt. Die Aufarbeitung erfolgt nach üblichen Methoden, beispielsweise durch Eingießen des Reaktionsgemisches in Wasser und Extrahieren mit einem mit Wasser praktisch nicht mischbaren Lösungsmittel, wie z.B. Toluol, und Abdestillieren des Extraktionsmittels unter vermindertem Druck.

Die Verbindungen der Formel (I g) sind bisher noch nicht bekannt. Sie lassen sich herstellen, indem man 2-Nitro-5-(4-trifluormethylphenoxy)-benzylhalogenide der Formel

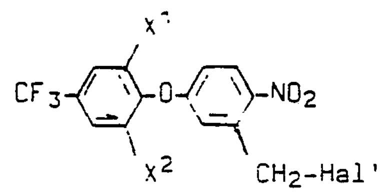

(VI)

in welcher

$X^1$ und $X^2$ und Hal' die oben angegebene Bedeutung haben,

mit Alkalixanthogenaten der Formel

$$M^3-S-\overset{\overset{S}{\|}}{C}-OR^6 \qquad (XIII)$$

in welcher

$R^6$ die oben angegebene Bedeutung hat und

$M^3$ für Natrium oder Kalium steht,

in Gegenwart eines Verdünnungsmittels, wie z.B. Tetrahydrofuran, bei Temperaturen zwischen -20 und +30°C umsetzt. Die Aufarbeitung erfolgt nach üblichen Methoden, beispielsweise durch Eingießen der Reaktionsmischung in Wasser, Extrahieren mit einem mit Wasser praktisch nicht mischbaren Lösungsmittel, wie z.B. Toluol, Waschen der Extraktionslösung mit Wasser und Abdestillieren des Extraktionsmittels unter vermindertem Druck.

Die als Ausgangsstoffe benötigten 2-Nitro-5-(4-trifluormethyl-phenoxy)-benzyl-halogenide der Formel (VI) sind bisher noch nicht bekannt. Sie lassen sich jedoch herstellen, indem man 2-Nitro-5-(4-trifluormethyl-phenoxy)-benzylalkohole der Formel

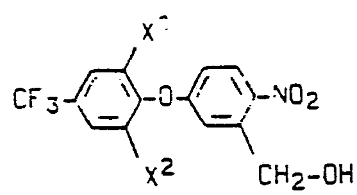

(IIa)

in welcher

$X^1$ und $X^2$ die oben angegebene Bedeutung haben,

gegebenenfalls in Gegenwart eines Katalysators, wie zum Beispiel p-Toluolsulfonsäure oder Dimethylformamid, sowie gegebenenfalls in Gegenwart eines Verdünnungsmittels, wie zum Beispiel Tetrahydrofuran, Toluol oder Ethylenchlorid, mit Stoffen, die zum Austausch von Hydroxy gegen Chlor oder Brom geeignet sind, bei Temperaturen zwischen $10^{o}C$ und $120^{o}C$ umsetzt.

Als Stoffe, die zum Ersatz von Hydroxy durch Chlor oder Brom geeignet sind, kommen vorzugsweise Thionylchlorid und Phosphortribromid in Frage.

Diejenigen Verbindungen der Formel (VI), in denen Hal' für Jod steht, lassen sich herstellen, indem man Verbindungen der Formel (VI), in denen Hal' für Chlor oder Brom steht, mit Alkaliiodiden, wie zum Beispiel Natriumiodid oder Kaliumiodid, in Gegenwart eines Verdünnungsmittels umsetzt.

Die Aufarbeitung erfolgt bei dem obigen Verfahren zur Herstellung der Verbindungen der Formel (VI) nach üblichen Methoden. Im allgemeinen geht man so vor, daß man das Reaktionsgemisch in einem mit Wasser praktisch nicht mischbaren organischen Lösungsmittel, wie Methylenchlorid, aufnimmt, die organische Phase mit Wasser wäscht, trocknet, filtriert und durch Abdestillieren des Lösungsmittels unter vermindertem Druck einengt.

Die bei dem erfindungsgemäßen Verfahren (b) weiterhin als Ausgangsstoffe benötigten Halogenverbindungen sind durch die Formel (V) definiert. In dieser Formel steht Hal für Chlor, Brom oder Iod. $Z^2$ steht vorzugsweise für gegebenenfalls durch Chlor substituiertes $C_3$-$C_5$-Alkenyl, $C_3$-$C_5$-Alkinyl, Benzyl, $C_1$-$C_4$-Alkyl oder Di-($C_1$-$C_4$)-alkylaminocarbonyl.

Als Beispiele für Verbindungen der Formel (V) seien genannt: Methylbromid, Methyliodid, Ethylchlorid, Ethylbromid, n-Propylbromid, iso-Propylchlorid, n-Butylchlorid, iso-Butylchlorid, Allylbromid, Propargylbromid, Benzylchlorid und Dimethylcarbamidsäurechlorid.

Als Säureakzeptoren und Verdünnungsmittel kommen bei der Durchführung des erfindungsgemäßen Verfahrens (b) vorzugsweise alle diejenigen Stoffe in Betracht, die bereits im Falle des erfindungsgemäßen Verfahrens (a) vorzugsweise als Säureakzeptoren bzw. Verdünnungsmittel genannt wurden.

Die Reaktionstemperaturen können bei dem erfindungsgemäßen Verfahren (b) ebenso variiert werden wie bei dem Verfahren (a). Auch die Durchführung der Umsetzung und die Aufarbeitung werden bei dem erfindungsgemäßen Verfahren (b) so durchgeführt, wie es für das erfindungsgemäße Verfahren (a) beschrieben wurde.

Die bei dem erfindungsgemäßen Verfahren (c) als Ausgangsstoffe benötigten 2-Nitro-5-(4-trifluormethylphenoxy)-benzyl-thioverbindungen sind durch die Formel (Ia) allgemein definiert. In dieser Formel stehen vorzugsweise

$X^1$ für Chlor,

$X^2$ für Wasserstoff oder Chlor und

$Z^3$ für Wasserstoff, $C_1$-$C_4$-Alkyl, $C_3$-$C_5$-Alkenyl, $C_3$-$C_5$-Alkinyl, Benzyl oder für gegebenenfalls durch Fluor, Chlor, Brom, Cyano, Nitro, $C_1$-$C_4$-Alkyl, Trifluormethyl, $C_1$-$C_4$-Alkoxy, $C_1$-$C_4$-Alkylthio, $C_1$-$C_4$-Alkylsulfinyl oder $C_1$-$C_4$-Alkylsulfonyl substituiertes Phenyl oder für Hydroxycarbonyl-($C_1$-$C_2$)-alkyl, $C_1$-$C_4$-Alkoxycarbonyl-$C_1$-$C_2$-alkyl oder den Rest der Formel

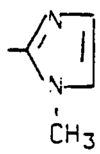 oder 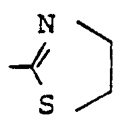 .

Als Beispiele für die Verbindungen der Formel (Ia) seien genannt: (2-Nitro-5-(2-chlor-4-trifluormethyl-phenoxy)-benzyl)- und (2-Nitro-5-(2,6-dichlor-4-trifluormethyl-phenoxy)-benzyl)-methylsulfid, -ethylsulfid, -n-propylsulfid, -iso-propylsulfid, -n-butylsulfid, -iso-butylsulfid, -allylsulfid, -propargylsulfid, -benzylsulfid, -phenylsulfid und -4-chlor-phenylsulfid.

Die Verbindungen der Formel (Ia) lassen sich nach einem oder mehreren der anderen hier beschriebenen erfindungsgemäßen Verfahren herstellen.

Das erfindungsgemäße Verfahren (c) wird unter Verwendung von Oxidationsmitteln, welche zur Herstellung von Disulfiden, Sulfoxiden und Sulfonen aus Thiolen und Sulfiden geeignet sind, durchgeführt. Als Beispiele für solche Oxidationsmittel seien Wasserstoffperoxid und m-Chlor-perbenzoesäure genannt.

Das erfindungsgemäße Verfahren (c) wird vorzugsweise in Gegenwart von Verdünnungsmitteln durchgeführt. Als Verdünnungsmittel kommen hierbei vorzugsweise diejenigen Solventien in Betracht, welche bereits im Zusammenhang mit der Beschreibung von Verfahren (a) genannt wurden. Ferner sind hierbei auch aliphatische Carbonsäuren, wie z.B. Essigsäure, als Verdünnungsmittel sehr gut geeignet.

Die Reaktionstemperaturen können auch beim erfindungsgemäßen Verfahren (c) in einem größeren Bereich variiert werden. Im allgemeinen arbeitet man bei Temperaturen zwischen -20 und +150°C, vorzugsweise zwischen 0 und 100°C.

Verfahren (c) wird im allgemeinen bei Normaldruck durchgeführt.

Zur Durchführung des erfindungsgemäßen Verfahrens (c) setzt man auf 1 Mol 2-Nitro-5-(4-trifluormethyl-phenoxy)-benzyl-thioether der Formel (Ia) zur Herstellung der entsprechenden Sulfoxide im allgemeinen zwischen 0,9 und 1,5 Mol, vorzugsweise 1,0 bis 1,2 Mol Oxidationsmittel und zur Herstellung der entsprechenden Sulfone zwischen 2 und 5 Mol, vorzugsweise zwischen 2,2 und 3 Mol Oxidationsmittel ein. Die Ausgangsstoffe der Formel (Ia) werden vorzugsweise in einem Verdünnungsmittel vorgelegt und langsam mit dem Oxidationsmittel versetzt; dann wird weiter bis zum Ende der Umsetzung gerührt. Die Aufarbeitung wird nach üblichen Methoden durchgeführt. Vorzugsweise geht man so vor, wie es oben für Verfahren (a) angegeben wurde.

Die bei dem erfindungsgemäßen Verfahren (d) als Ausgangsstoffe benötigten 2-Nitro-5-(4-trifluormethyl-phenoxy)-benzylhalogenide sind durch die Formel (VI) definiert. In dieser Formel stehen vorzugsweise

$X^1$ für Chlor,

$X^2$ für Wasserstoff oder Chlor und

Hal' für Chlor oder Brom.

Die Verbindungen der Formel (VI) und deren Herstellung wurden bereits oben im Zusammenhang mit der Beschreibung des Verfahrens (b) erwähnt.

Die beim erfindungsgemäßen Verfahren (d), Variante $\alpha$, weiter als Ausgangsstoffe zu verwendenden Nucleophile sind durch die Formel (VII) allgemein definiert. In dieser Formel steht vorzugsweise

$M^1$ für Wasserstoff, Natrium oder Kalium und

Z hat vorzugsweise diejenigen Bedeutungen, die im Zusammenhang mit der Beschreibung der erfindungsgemäßen Verbindungen der Formel (I) vorzugsweise für diesen Rest angegeben wurden.

Als Beispiele für die Verbindungen der Formel (VII) seien genannt: Allylalkohol, Benzylalkohol, Propargylalkohol, Natriummethylmercaptid, Kaliumthiophenolat,

Mercaptoessigsäure, Mercaptoessigsäuremethylester, Mercaptoessigsäureethylester, Mercaptoessigsäuredimethylamid, Ammoniak, Methylamin, Ethylamin, n-Butylamin, Cyclopentylamin, Benzylamin, Dimethylamin, Diethylamin, Diallylamin, Dipropargylamin, Bis-(2-chlorethyl)-amin, Bis-(2-cyanoethyl)-amin, Bis-(2-methoxyethyl)-amin, Aminoessigsäure, Aminoessigsäuremethylester, Aminoessigsäureethylester, N-Methyl-anilin, N-Ethyl-anilin, N-Propylanilin, Pyrrolidin, 2-Methyl- und 2-Ethyl-pyrrolidin, Piperidin, 2-Methyl-, 3-Methyl-, 4-Methyl-, 2,4-Dimethyl-, 2-Ethyl- und 2-Methyl-5-ethyl-piperidin, Morpholin und Hexamethylenimin sowie Natrium-0,0-dimethyl-dithiophosphat und Natrium-0,0-diethyldithiophosphat.

Die Ausgangsverbindungen der Formel (VII) sind bekannt.

Das erfindungsgemäße Verfahren (d), Variante $\alpha$, wird vorzugsweise in Gegenwart von Verdünnungsmitteln durchgeführt. Als Verdünnungsmittel kommen hierbei vorzugsweise diejenigen Solventien in Betracht, welche bereits im Zusammenhang mit der Beschreibung von Verfahren (a) genannt wurden.

Als Säureakzeptoren, welche bei Verfahren (d), Variante $\alpha$, gegebenenfalls zu verwenden sind, kommen vorzugsweise diejenigen Säurebindemittel in Betracht, welche bereits im Zusammenhang mit der Beschreibung von Verfahren (a) genannt wurden, ferner auch Alkoholate, wie z.B. Natriummethylat und Natriumethylat.

Die Reaktionstemperaturen können auch beim erfindungsgemäßen Verfahren (d), Variante α, in einem größeren Bereich variiert werden. Im allgemeinen arbeitet man bei Temperaturen zwischen -20 und +150°C, vorzugsweise zwischen 0 und 100°C.

Verfahren (d), Variante α, wird im allgemeinen bei Normaldruck durchgeführt.

Zur Durchführung des erfindungsgemäßen Verfahrens (d), Variante α, setzt man auf 1 Mol 2-Nitro-5-(4-trifluormethyl-phenoxy)-benzylhalogenid der Formel (VI) im allgemeinen zwischen 1 und 10 Mol, vorzugsweise 1 bis 5 Mol Nucleophil der Formel (VII) ein. Die Ausgangsstoffe werden vorzugsweise unter Zusatz eines Verdünnungsmittels und gegebenenfalls unter Zusatz eines Säureakzeptors vermischt und gegebenenfalls bei erhöhter Temperatur bis zum Ende der Umsetzung gerührt. Die Aufarbeitung wird nach üblichen Methoden durchgeführt. Vorzugsweise geht man so vor, wie es oben für Verfahren (a) angegeben wurde.

Die beim erfindungsgemäßen Verfahren (d), Variante ß, als Reaktionskomponenten zu verwendenden Trialkylphosphite sind durch die Formel (VIII) allgemein definiert. In dieser Formel steht vorzugsweise

R für Methyl oder Ethyl.

Als Beispiele für die Verbindungen der Formel (VIII) seien Trimethylphosphit und Triethylphosphit genannt.

Die Trialkylphosphite der Formel (VIII) sind bekannt.

Das erfindungsgemäße Verfahren (d), Variante ß, wird gegebenenfalls in Gegenwart von Verdünnungsmitteln durchgeführt. Als Verdünnungsmittel kommen hierbei vorzugsweise diejenigen Solventien in Betracht, welche bereits im Zusammenhang mit der Beschreibung von Verfahren (a) genannt wurden.

Als Katalysatoren, welche gegebenenfalls beim erfindungsgemäßen Verfahren (d), Variante ß, eingesetzt werden, seien Alkaliiodide, wie z.B. Natriumiodid und Kaliumiodid genannt. Es werden gegebenenfalls weitere Katalysatoren, wie z.B. der Kronenether 15-Krone-5 verwendet.

Die Reaktionstemperaturen können auch beim erfindungsgemäßen Verfahren (d), Variante ß, in einem größeren Bereich variiert werden. Im allgemeinen arbeitet man bei Temperaturen zwischen 0 und 250°C, vorzugsweise zwischen 10 und 200°C.

Das Verfahren (d), Variante ß, wird im allgemeinen bei Normaldruck oder bei erhöhtem Druck bis etwa 10 bar durchgeführt.

Zur Durchführung des erfindungsgemäßen Verfahrens (d), Variante ß, setzt man auf 1 Mol 2-Nitro-5-(4-trifluormethyl-phenoxy)-benzylhalogenid der Formel (VI) im allgemeinen zwischen 1 und 2 Mol, vorzugsweise zwischen 1,1 und 1,5 Mol Trialkylphosphit der Formel (VIII) ein. Die Ausgangsstoffe werden vorzugsweise unter Zusatz eines Verdünnungsmittels und gegebenenfalls unter Zusatz von Katalysatoren vermischt und gegebenenfalls bei erhöhter Temperatur bis zum Ende der Umsetzung gerührt. Die Aufarbeitung wird nach üblichen Methoden durchgeführt. Vorzugsweise geht man so vor, wie es oben für Verfahren (a) angegeben wurde.

Die bei Verfahren (d), Variante ß, entstehenden Phosphonsäureester der Formel (Ib) können nach üblichen Methoden, beispielsweise durch Erhitzen mit wäßrigen Protonensäuren, wie z.B. Salzsäure und/oder Essigsäure, verseift werden. Eine besonders schonende Verseifungsmethode besteht darin, daß man die Phosphonsäureester der Formel (Ib) mit Trimethylchlorsilan, gegebenenfalls in Gegenwart eines Katalysators, wie z.B. Natriumiodid, und in Gegenwart eines Verdünnungsmittels, wie z.B. Acetonitril, bei Temperaturen zwischen 0 und 50°C umsetzt und anschließend mit Wasser, gegebenenfalls unter Zusatz einer Säure, wie z.B. Salzsäure, verrührt. Die kristallinen Phosphonsäuren können dann durch Absaugen isoliert werden. Soweit die Phosphonsäuren in öliger Form anfallen, können sie auf übliche Weise isoliert werden. Im allgemeinen verfährt man so, wie es im Falle des erfindungsgemäßen Verfahrens (a) beschrieben wurde.

Aus den Phosphonsäuren der Formel (Ic) erhält man entsprechende Natrium-, Kalium- oder Calciumsalze durch Umsetzung mit Natriumverbindungen, wie z. B. Natriumhydroxid, Kaliumverbindungen, wie z. B. Kaliumhydroxid bzw. Calciumverbindungen, wie z. B. Calciumchlorid und/oder Calciumhydroxid, in Gegenwart eines Verdünnungsmittels, wie z. B. Wasser und Methanol, bei Temperaturen zwischen 0 und 50°C. Die Salze fallen kristallin an und können durch Absaugen isoliert werden.

Die bei dem erfindungsgemäßen Verfahren (e) als Ausgangsstoffe benötigten 2-Nitro-5-(4-trifluormethyl-phenoxy)-benzylamine sind durch die Formel (Id) allgemein definiert. In dieser Formel haben vorzugsweise $X^1$, $X^2$, $R^1$ und $R^2$ diejenigen Bedeutungen, die bereits im Zusammenhang mit der Beschreibung der erfindungsgemäßen Verbindungen der Formel (I) vorzugsweise für diese Reste genannt wurden.

Als Beispiele für die Verbindungen der Formel (Id) seien genannt: N-(2-Nitro-5-(2-chlor-4-trifluormethyl-phenoxy)-benzyl)- und N-(2-Nitro-5-(2,6-dichlor-4-trifluormethyl-phenoxy)-benzyl)-dimethylamin, -diethylamin, -dipropylamin, -bis-(2-chlorethyl)-amin, -pyrrolidin, -piperidin, -4-methyl-piperidin, -morpholin, -pyrazol, -imidazol und -1,2,4-triazol.

Die Verbindungen der Formel (Id) sind bisher noch nicht bekannt; sie lassen sich jedoch nach einem oder mehreren der anderen erfindungsgemäßen Verfahren in einfacher Weise herstellen.

Die bei dem erfindungsgemäßen Verfahren (e) weiter als Ausgangsstoffe zu verwendenden Halogenide sind durch die Formel (X) allgemein definiert. In dieser Formel steht

Hal" für Chlor, Brom oder Iod und

$R^3$ steht vorzugsweise für Wasserstoff oder $C_1$-$C_4$-Alkyl.

Als Beispiele für Verbindungen der Formel (X) seien genannt: Chlorwasserstoff, Bromwasserstoff, Iodwasserstoff, Methyliodid, Ethyliodid, Ethylbromid, Ethylchlorid, n-Propylbromid, iso-Propylchlorid, n-Butylbromid und n-Butylchlorid.

Die Verbindungen der Formel (X) sind bekannt.

Das erfindungsgemäße Verfahren (e) wird vorzugsweise in Gegenwart von Verdünnungsmitteln durchgeführt. Als Verdünnungsmittel kommen hierbei insbesondere diejenigen Solventien in Betracht, welche bereits im Zusammenhang mit der Beschreibung von Verfahren (a) genannt wurden.

Die Reaktionstemperaturen können auch beim erfindungsgemäßen Verfahren (e) in einem größeren Bereich variiert werden. Im allgemeinen arbeitet man bei Temperaturen zwischen 0 und 150°C, vorzugsweise zwischen 10 und 100°C.

Das Verfahren (e) wird im allgemeinen bei Normaldruck oder bei erhöhtem Druck bis etwa 10 bar durchgeführt.

Zur Durchführung des erfindungsgemäßen Verfahrens (e) setzt man auf 1 Mol 2-Nitro-5-(4-trifluormethyl-phenoxy)-benzylamin der Formel (Id) im allgemeinen zwischen 1 und 3 Mol, vorzugsweise zwischen 1 und 2 Mol Halogenid der Formel (X) ein. Die Ausgangsstoffe werden vorzugsweise unter Zusatz eines Verdünnungsmittels vermischt und gegebenenfalls bei erhöhter Temperatur bis zum Ende der Umsetzung gerührt. Die Produkte fallen nach Abkühlen in kristalliner Form an und können durch Absaugen isoliert werden.

Die bei dem erfindungsgemäßen Verfahren (f) als Ausgangsstoffe benötigten substituierten 2-Nitro-5-(4-trifluormethyl-phenoxy)-toluole sind durch die Formel (Ie) allgemein definiert. In dieser Formel stehen vorzugsweise

$X^1$ für Chlor,

$X^2$ für Wasserstoff oder Chlor und

$Z^4$ für $C_1$-$C_4$-Alkoxy-carbonyl-$C_1$-$C_2$-alkylthio oder $C_1$-$C_4$-Alkoxy-carbonyl-$C_1$-$C_2$-alkylamino.

Als Beispiele für Verbindungen der Formel (XI) seien genannt:
α-(Methoxycarbonylmethylthio)-, α-(Ethoxycarbonylmethylthio)-, α-(1-Methoxycarbonyl-ethylthio)-, α-(1-Ethoxycarbonyl-ethylthio)-, α-(Methoxycarbonylmethylamino)-, α-(Ethoxycarbonylmethylamino)-, α-(1-Methoxycarbonyl-ethylamino)- und α-(1-Ethoxycarbonyl-ethylamino)-
-2-nitro-5-(2-chlor-4-trifluormethyl-phenoxy)-toluol und -2-nitro-5-(2,6-dichlor-4-trifluormethyl-phenoxy)-toluol.

Die Verbindungen der Formel (Ie) sind bisher noch nicht bekannt; sie lassen sich jedoch nach einem oder mehreren der anderen erfindungsgemäßen Verfahren in einfacher Weise herstellen.

Als Alkalihydroxide, welche beim erfindungsgemäßen Verfahren (f) als Reaktionskomponenten zu verwenden sind, seien Natriumhydroxid und Kaliumhydroxid genannt. Als Verdünnungsmittel dient Wasser.

Als Mineralsäuren, welche beim erfindungsgemäßen Verfahren (f) weiter als Reaktionskomponenten zu verwenden sind, seien Salzsäure und Schwefelsäure genannt.

Die Reaktionstemperaturen können bei der Durchführung des erfindungsgemäßen Verfahrens (f) innerhalb eines größeren Bereichs variiert werden. Im allgemeinen arbeitet man zwischen 0 und 100°C, vorzugsweise zwischen 10 und 50°C. Das erfindungsgemäße Verfahren (f) wird im allgemeinen bei Normaldruck durchgeführt.

Zur Durchführung des erfindungsgemäßen Verfahrens (f) setzt man auf 1 Mol Ausgangsverbindung der Formel (Ie) im allgemeinen 1 bis 2 Mol, vorzugsweise 1 bis 1,2 Mol, Alkalihydroxid enthaltende Alkalilauge ein. Die Verbindung der Formel (Ie) wird in der Alkalilauge dispergiert und gegebenenfalls bei erhöhter Temperatur bis zum Ende der Umsetzung gerührt. Dann wird eine Mineralsäure zum Reaktionsgemisch gegeben, bis ein pH-Wert zwischen 1 und 5 eingestellt ist. Die Isolierung der auf diese Weise entstandenen Carbonsäuren erfolgt nach üblichen Methoden, beispielsweise so, wie es bei den vorausgehend beschriebenen erfindungsgemäßen Verfahren erwähnt wurde.

Die bei dem erfindungsgemäßen Verfahren (g) als Ausgangsstoffe benötigten 3-(4-Trifluormethyl-phenoxy)-toluole sind durch die Formel (XI) allgemein definiert. In dieser Formel haben vorzugsweise $X^1$, $X^2$ und Z diejenigen Bedeutungen, die bereits im Zusammenhang mit der Beschreibung der erfindungsgemäßen Verbindungen der Formel (I) vorzugsweise für diese Reste genannt wurden.

Als Beispiele für die Verbindungen der Formel (XI) seien genannt:

α-Acetoxy-, α-Chloracetoxy-, α-Allyloxy, α-Propargyloxy-, α-Methylthio-, α-Ethylthio, α-n-Propylthio-, α-iso-Propylthio-, α-Dimethylamino-, α-Diethylamino-, α-Pyrazolyl-, α-Hydroxycarbonylmethylthio-, α-Methoxycarbonylmethylthio-, α-Hydroxycarbonylmethylsulfinyl-, α-Methoxycarbonylmethylsulfinyl-, α-Hydroxycarbonylmethyl-sulfonyl-, α-Methoxycarbonylmethylsulfonyl-, α-Dimethylaminocarbonylthio-, α-Diethylaminocarbonylthio-, α-Methoxycarbonyloxy-, α-Ethoxycarbonyloxy-, α-Methylsulfinyl-, α-Ethylsulfinyl-, α-Methylsulfonyl-, α-Ethylsulfonyl-, α-Triazolyl-, α-(N-Methyl-N-phenyl-amino)-, α-Morpholinyl-, α-(2-Methyl-piperidyl)-, α-Hydroxycarbonylmethylamino- und α-Methoxycarbonylmethylamino-3-(2-chlor-4-trifluormethyl-phenoxy)-toluol und 3-(2,6-dichlor-4-trifluormethyl-phenoxy)-toluol sowie 3-(2-Chlor-4-trifluormethyl-phenoxy)-benzyl- und 3-(2,6-Dichlor-4-trifluormethyl-phenoxy)-benzylphosphonsäure, deren Methylester und deren Ethylester.

Die Verbindungen der Formel (XI) sind bisher noch nicht bekannt; sie lassen sich jedoch analog den erfindungsgemäßen Verfahren (a) bis (f) in einfacher Weise herstellen.

Im einzelnen lassen sich die Verbindungen der Formel (XI) herstellen, indem man

(h) 3-(4-Trifluormethyl-phenoxy)-benzylalkohole der Formel

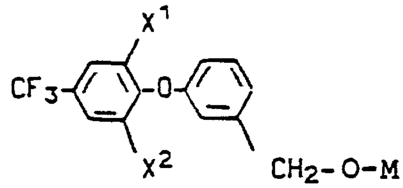

(XIV)

in welcher

$X^1$, $X^2$ und M die oben angegebene Bedeutung haben,

mit Halogenverbindungen der Formel

$$Hal-Z^1 \quad (III)$$

in welcher

Hal und $Z^1$ die oben angegebene Bedeutung haben,

gegebenenfalls in Gegenwart eines Säureakzeptors und gegebenenfalls in Gegenwart eines Verdünnungsmittels umsetzt,

oder

(i) 3-(4-Trifluormethyl-phenoxy)-benzylthiole der Formel

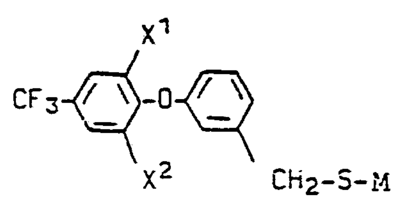

(XV)

in welcher

$X^1$, $X^2$ und M die oben angegebene Bedeutung haben,

mit Halogenverbindungen der Formel

$$Hal-Z^2 \qquad (V)$$

in welcher

Hal und $Z^2$ die oben angegebene Bedeutung haben,

gegebenenfalls in Gegenwart eines Säureakzeptors und gegebenenfalls in Gegenwart eines Verdünnungsmittels umsetzt,

oder

(j) 3-(4-Trifluormethyl-phenoxy)-benzylthioverbindungen der Formel

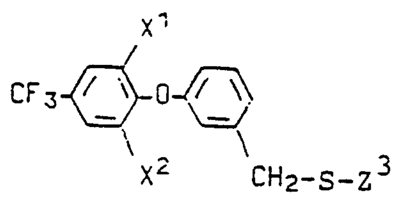

(XIa)

in welcher

$X^1$, $X^2$ und $Z^3$ die oben angegebene Bedeutung haben,

mit Oxidationsmitteln gegebenenfalls in Gegenwart eines Verdünnungsmittels umsetzt,

oder

(k) 3-(4-Trifluormethyl-phenoxy)-benzylhalogenide der Formel

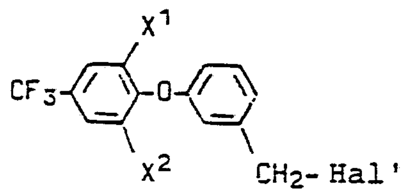

(XVI)

in welcher

$X^1$, $X^2$ und Hal' die oben angegebene Bedeutung haben,

α) mit Nucleophilen der Formel

$$M^1-Z \quad (VII)$$

in welcher

$M^1$ und Z die oben angegebene Bedeutung haben,

gegebenenfalls in Gegenwart eines Säureakzeptors sowie gegebenenfalls in Gegenwart eines Verdünnungsmittels umsetzt,

oder

β) mit Trialkylphosphiten der Formel

$P(OR)_3$ (VIII)

in welcher

R für Alkyl steht,

gegebenenfalls in Gegenwart eines Katalysators und gegebenenfalls in Gegenwart eines Verdünnungsmittels umsetzt und gegebenenfalls anschließend die dabei entstehenden Phosphonsäureester der Formel

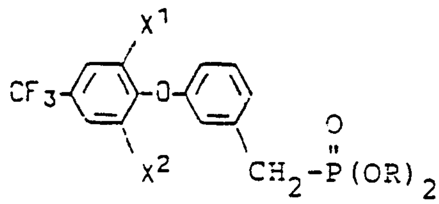
(XIb)

in welcher

$X^1$, $X^2$ und R die oben angegebene Bedeutung haben,

gegebenenfalls in Gegenwart eines Verdünnungsmittels verseift und gegebenenfalls die dabei entstehenden Säuren der Formel

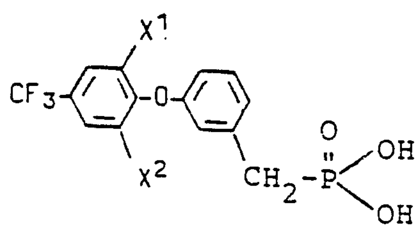
(XIc)

in welcher

$X^1$ und $X^2$ die oben angegebene Bedeutung haben,

mit einer Verbindung der Formel

$$M^2X^3 \qquad (IX)$$

in welcher

$M^2$ und $X^3$ die oben angegebene Bedeutung haben,

gegebenenfalls in Gegenwart eines Verdünnungsmittels umsetzt,

oder

(l) 3-(4-Trifluormethyl-phenoxy)-benzylamine der Formel

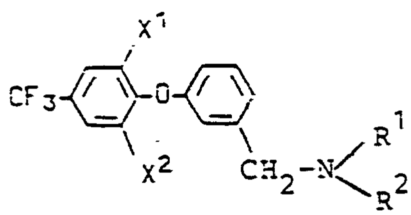

(XId)

in welcher

$X^1$, $X^2$, $R^1$ und $R^2$ die oben angegebene Bedeutung haben,

mit Halogeniden der Formel

$$Hal''-R^3 \qquad (X)$$

in welcher

Hal" und $R^3$ die oben angegebene Bedeutung haben,

gegebenenfalls in Gegenwart eines Verdünnungsmittels umsetzt,

oder

(m) substituierte 3-(4-Trifluormethyl-phenoxy)-toluole der Formel

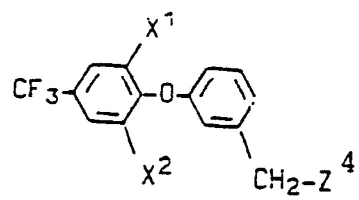

(XIe)

in welcher

$X^1$, $X^2$ und $Z^4$ die oben angegebene Bedeutung haben,

mit Alkalihydroxiden in Gegenwart eines Verdünnungsmittels umsetzt und die dabei entstehenden Carbonsäuresalze mit Mineralsäuren behandelt.

Die bei den Verfahren (h) bis (k) als Ausgangsstoffe benötigten Verbindungen (XIV), (XV) und (XVI) sind teilweise bekannt. Die bisher noch nicht bekannten Stoffe lassen sich jedoch nach im Prinzip bekannten Methoden in einfacher Weise herstellen.

Bei den Verfahren (h) bis (k) entsprechen die Umsetzungsbedingungen denjenigen der analogen erfindungsgemäßen Verfahren (a) bis (f).

Das erfindungsgemäße Verfahren (g) wird gegebenenfalls unter Verwendung eines Verdünnungsmittels durchgeführt. Als Verdünnungsmittel kommen hierbei vorzugsweise halogenierte Kohlenwasserstoffe, wie z.B. Methylenchlorid, in Betracht.

Das erfindungsgemäße Verfahren (g) wird gegebenenfalls in Gegenwart eines Katalysators durchgeführt. Als Katalysator kommen vorzugsweise Protonensäuren, wie z.B. Schwefelsäure oder Essigsäure, in Betracht.

Die Reaktionstemperatur wird bei Verfahren (g) im allgemeinen zwischen -20 und +80°C, vorzugsweise zwischen 0 und 50°C gehalten. Verfahren (g) wird im allgemeinen bei Normaldruck durchgeführt.

Zur Durchführung des erfindungsgemäßen Verfahrens (g) setzt man auf 1 Mol Ausgangsverbindung der Formel (XI) im allgemeinen zwischen 0,9 und 2 Mol, vorzugsweise 1,0 bis 1,3 Mol konzentrierte Salpetersäure und gegebenenfalls etwa die gleiche Menge eines Katalysators ein. Die Ausgangskomponenten werden vorzugsweise unter Eiskühlung zusammengegeben und dann gegebenenfalls bei leicht erhöhter Temperatur bis zum Ende der Umsetzung gerührt. Die Aufarbeitung erfolgt nach üblichen Methoden. Im allgemeinen geht man so vor, wie es bereits im Zusammenhang mit den vorausgehend beschriebenen erfindungsgemäßen Verfahren erwähnt wurde.

Die erfindungsgemäßen Wirkstoffe beeinflussen das Pflanzenwachstum und können deshalb als Defoliants, Desiccants, Krautabtötungsmittel, Keimhemmungsmittel und insbesondere als Unkrautvernichtungsmittel verwendet werden. Unter Unkraut im weitesten Sinne sind alle Pflanzen zu verstehen, die an Orten aufwachsen, wo sie unerwünscht sind. Ob die erfindungsgemäßen Stoffe als totale oder selektive Herbizide wirken, hängt im wesentlichen von der angewendeten Menge ab.

Die erfindungsgemäßen Wirkstoffe können z.B. bei den folgenden Pflanzen verwendet werden:

Dikotyle Unkräuter der Gattungen: Sinapis, Lepidium, Galium, Stellaria, Matricaria, Anthemis, Galinsoga, Chenopodium, Urtica, Senecio, Amaranthus, Portulaca, Xanthium, Convolvulus, Ipomoea, Polygonum, Sesbania, Ambrosia, Cirsium, Carduus, Sonchus, Solanum, Rorippa, Rotala, Lindernia, Lamium, Veronica, Abutilon, Emex, Datura, Viola, Galeopsis, Papaver, Centaurea.

Dicotyle Kulturen der Gattungen: Gossypium, Glycine, Beta, Daucus, Phaseolus, Pisum, Solanum, Linum, Ipomoea, Vicia, Nicotiana, Lycopersicon, Arachis, Brassica, Lactuca; Cucumis, Cucurbita.

Monokotyle Unkräuter der Gattungen: Echinochloa, Setaria, Panicum, Digitaria, Phleum, Poa, Festuca, Eleusine, Brachiaria, Lolium, Bromus, Avena, Cyperus, Sorghum, Agropyron, Cynodon, Monochoria, Fimbristylis, Sagittaria, Eleocharis, Scirpus, Paspalum, Ischaemum, Sphenoclea, Dactyloctenium, Agrostis, Alopecurus, Apera.

Monokotyle Kulturen der Gattungen: Oryza, Zea, Triticum, Hordeum, Avena, Secale, Sorghum, Panicum, Saccharum, Ananas, Asparagus, Allium.

Die Verbindungen eignen sich in Abhängigkeit von der Konzentration zur Totalunkrautbekämpfung z.B. auf Industrie- und Gleisanlagen und auf Wegen und Plätzen mit und ohne Baumbewuchs. Ebenso können die Verbindungen zur Unkrautbekämpfung in Dauerkulturen z.B. Forst-, Ziergehölz-, Obst-, Wein-, Citrus-, Nuss-, Bananen-, Kaffee-, Tee-, Gummi-, Ölpalm-, Kakao-, Beerenfrucht- und Hopfenanlagen und zur selektiven Unkrautbekämpfung in einjährigen Kulturen eingesetzt werden.

Die erfindungsgemäßen Wirkstoffe eignen sich insbesondere zur selektiven Bekämpfung von Unkräutern in wichtigen Kulturen, wie zum Beispiel Mais und Baumwolle.

Die erfindungsgemäßen Wirkstoffe besitzen außerdem eine sehr gute pflanzenwachstumsregulierende Wirksamkeit. Sie eignen sich besonders gut zur Wuchshemmung, sowie zur Entlaubung und Austrocknung der Blätter bei Baumwolle und zur Kartoffelkrautabtötung.

Die Wirkstoffe können in die üblichen Formulierungen überführt werden, wie Lösungen, Emulsionen, Spritzpulver, Suspensionen, Pulver, Stäubemittel, Pasten, lösliche Pulver, Granulate, Suspension-Emulsions-Konzentrate, wirkstoffimprägnierte Natur- und synthetische Stoffe, Feinstverkapselungen in polymeren Stoffen.

Diese Formulierungen werden in bekannter Weise hergestellt, z.B. durch Vermischen der Wirkstoffe mit Streckmitteln, also flüssigen Lösungsmitteln und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten und chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol

oder Glykol sowie deren Ether und Ester, Ketone wie Aceton, Methylethylketon, Methylisobutylketon oder Cyclohexanon, stark polare Lösungsmittel, wie Dimethylformamid und Dimethylsulfoxid, sowie Wasser.

Als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silicate; als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengeln; als Emulgier- und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylaryl-polyglykolether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können als solche oder in ihren Formulierungen auch in Mischung mit bekannten Herbiziden oder Pflanzenwachstumsregulatoren zur Unkrautbekämpfung bzw. als Pflanzenwuchsregulatoren Verwendung finden, wobei Fertigformulierungen oder Tankmischungen möglich sind. Auch eine Mischung mit anderen bekannten Wirkstoffen, wie Fungiziden, Insektiziden, Akariziden, Nematiziden, Schutzstoffen gegen Vogelfraß, Wuchsstoffen, Pflanzennährstoffen und Bodenstrukturverbesserungsmitteln ist möglich.

Die Wirkstoffe können als solche, in Form ihrer Formulierungen oder der daraus durch weiteres Verdünnen bereiteten Anwendungsformen, wie gebrauchsfertige Lösungen, Suspensionen, Emulsionen, Pulver, Pasten und Granulate angewandt werden. Die Anwendung geschieht in üblicher Weise, z.B. durch Gießen, Spritzen, Sprühen, Streuen.

Die erfindungsgemäßen Wirkstoffe können bei einer Anwendung als Herbizide sowohl vor als auch nach dem Auflaufen der Pflanzen appliziert werden. Die Anwendung wird vorzugsweise vor dem Auflaufen der Pflanzen, also im preemergence-Verfahren, vorgenommen. Sie können auch vor der Saat in den Boden eingearbeitet werden.

Für die Anwendung der erfindungsgemäßen Wirkstoffe als Pflanzenwachstumsregulatoren gilt, daß die Anwendung in einem bevorzugten Zeitraum vorgenommen wird, dessen genaue Abgrenzung sich nach den klimatischen und vegetativen Gegebenheiten richtet.

Die angewandte Wirkstoffmenge kann in einem größeren Bereich schwanken. Sie hängt im wesentlichen ab von der Art des gewünschten Effektes. Im allgemeinen liegen die Aufwandmengen zwischen 0,01 und 50 kg Wirkstoff pro Hektar Bodenfläche, vorzugsweise zwischen 0,05 und 10 kg pro Ha.

Die Herstellung und die Verwendung der erfindungsgemäßen Wirkstoffe gehen aus den nachfolgenden Beispielen hervor.

Herstellungsbeispiele

Beispiel 1

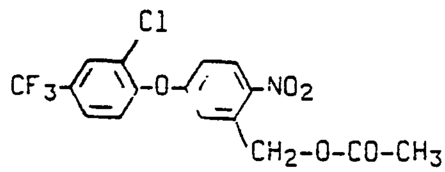

Eine Lösung von 5,1 g (0,065 Mol) Acetylchlorid in 20 ml Toluol wird zu einer auf 0 bis 5°C gekühlten Mischung aus 17,4 g (0,05 Mol) 2-Nitro-5-(2-chlor-4-trifluormethyl-phenoxy)-benzylalkohol, 6,1 g Triethylamin und 100 ml Toluol tropfenweise gegeben. Dann wird das Gemisch etwa 15 Stunden bei 20°C gerührt. Nach dem Verdünnen mit Toluol wird die organische Phase mit Wasser gewaschen, mit Natriumsulfat getrocknet, filtriert, und vom Filtrat wird das Lösungsmittel unter vermindertem Druck sorgfältig abdestilliert. Man erhält 16,9 g (87 % der Theorie) Essigsäure-(2-nitro-5-(2-chlor-4-trifluormethyl-phenoxy)-benzyl)-ester als gelbes Öl vom Brechungsindex $n_D^{20}$: 1,5488.

Beispiel 2

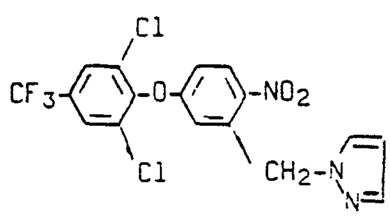

Eine Mischung aus 20 g (0,05 Mol) 2-Nitro-5-(2,6-dichlor-4-trifluormethyl-phenoxy)-benzylchlorid, 5,7 g

(0,084 Mol) Pyrazol, 12 g Kaliumcarbonat und 80 ml Acetonitril wird etwa 12 Stunden unter Rückfluß zum Sieden erhitzt, dann in 1 l Wasser gegossen, abgesaugt und neutral gewaschen. Das kristalline Rohprodukt wird mit Diethylether verrührt und abgesaugt. Man erhält 12,3 g (57 % der Theorie) 1-(2-Nitro-5-(2,6-dichlor-4-trifluormethyl-phenoxy)-benzyl)-pyrazol in Form beigefarbener Kristalle vom Schmelzpunkt 136°C.

Beispiel 3

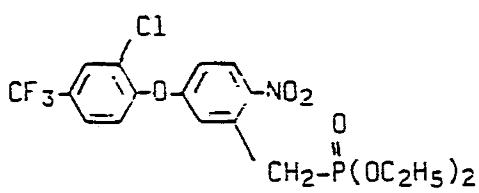

9,1 g (0,55 Mol) Triethylphosphit werden bei 20°C zu einer Mischung aus 18,3 g (0,05 Mol) 2-Nitro-5-(2-chlor-4-trifluormethyl-phenoxy)-benzylchlorid, 8,2 g (0,55 Mol) Natriumiodid, 1 g 15-Krone-5 und 80 ml Aceton gegeben, und das Reaktionsgemisch wird 7 Stunden unter Rückfluß zum Sieden erhitzt. Nach Zugabe von weiteren 1,7 g (0,01 Mol) Triethylphosphit und 1,5 g (0,01 Mol) Natriumiodid wird weitere 5 Stunden erhitzt, dann eingeengt, mit Toluol verdünnt, mit 2 n Salzsäure und mit Wasser gewaschen, getrocknet und filtriert, und vom Filtrat wird das Lösungsmittel unter vermindertem Druck sorgfältig abdestilliert. Man erhält 19,3 g (83 % der Theorie) 2-Nitro-5-(2-chlor-4-trifluormethyl-phenoxy)-benzyl-phosphonsäurediethylester als orangefarbenes Öl vom Brechungsindex $n_D^{20}$: 1,5311.

Beispiel 4

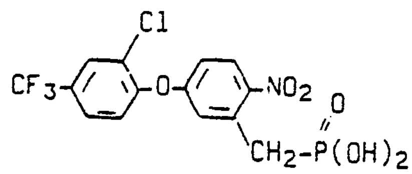

23,9 g (0,22 Mol) Trimethylchlorsilan werden zu einer Mischung aus 51,4 g (0,11 Mol) 2-Nitro-5-(2-chlor-4-trifluormethyl-phenoxy)-benzyl-phosphonsäurediethylester, 33 g (0,22 Mol) Natriumiodid und 110 ml Acetonitril gegeben, und die Mischung wird 8 Stunden bei 40°C gerührt. Nach dem Einengen wird der Rückstand mit 250 ml Wasser verrührt und mit etwas Natriumboranat versetzt. Nach 90 Minuten Rühren bei 40°C wird mit 5 ml konz. Salzsäure angesäuert und mit Methylenchlorid extrahiert. Der Extrakt wird mit Wasser gewaschen, getrocknet und filtriert. Das Filtrat wird durch Abdestillieren des Lösungsmittels unter vermindertem Druck eingeengt. Man erhält 42,2 g (93 % der Theorie) 2-Nitro-5-(2-chlor-4-trifluormethyl-phenoxy)-benzyl-phosphonsäure als hochviskoses orangefarbenes Öl.

Beispiel 5

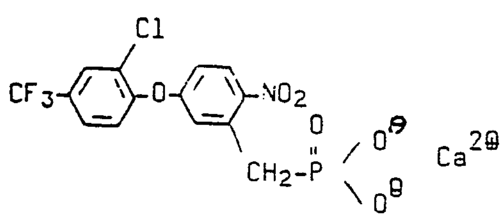

Eine Mischung aus 12,3 g (0,03 Mol) 2-Nitro-5-(2-chlor-4-trifluor-phenoxy)-benzyl-phosphonsäure und 30 ml Me-

thanol wird bei 20°C mit einer Lösung von 2,6 g Natriumhydroxid in 50 ml Wasser und dann mit einer Lösung von 7,2 g Calciumchlorid in 50 ml Wasser versetzt. Das ausfallende Produkt wird abgesaugt, mit Wasser gewaschen und getrocknet. Man erhält 10,0 g (75 % der Theorie) 2-Nitro-5-(2-chlor-4-trifluormethyl-phenoxy)-benzyl-phosphonsäure-Calciumsalz in Form elfenbeinfarbiger Kristalle.

Beispiel 6

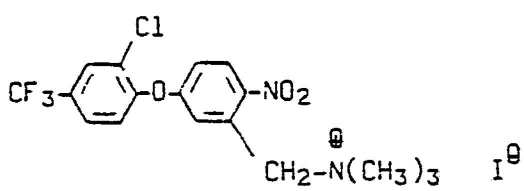

Eine Mischung aus 26,5 g (0,07 Mol) N,N-Dimethyl-(2-nitro-5-(2-chlor-4-trifluormethyl-phenoxy)-benzyl)-amin, 14,9 g Methyliodid und 100 ml Cyclohexan wird 6 Stunden bei 40°C gerührt. Nach Zugabe von weiteren 11,4 g Methyliodid wird weitere 2 Stunden bei 40°C gerührt. Das ausfallende Produkt wird abgesaugt, mit Cyclohexan gewaschen und aus Isopropanol umkristallisiert. Man erhält 8,2 g (23 % der Theorie) N,N,N-Trimethyl-(2-nitro-5-(2-chlor-4-trifluormethyl-phenoxy)-benzyl)-ammoniumiodid in Form gelber Kristalle vom Schmelzpunkt 185°C.

Beispiel 7

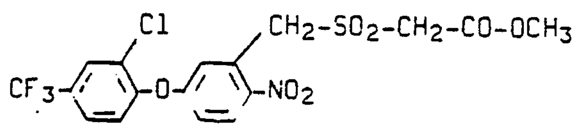

In eine Lösung von 21,8 g (0,05 Mol) 2-Nitro-5-(2-chlor-4-trifluormethyl-phenoxy)-benzylmercaptoessigsäuremethylester und 60 ml Essigsäure werden bei 75 - 80°C 14,8 g (0,13 Mol) einer 30 %igen Wasserstoffperoxidlösung getropft. Die Reaktionsmischung wird in 1 l Wasser gegossen, und die organische Substanz wird mit Toluol extrahiert. Die Toluolphase wird einmal mit 200 ml einer 10 %igen wäßrigen Natriumhydrogencarbonat-Lösung gewaschen und dann eingeengt. Der ölige Rückstand wird dann mit 50 ml eines Gemisches aus 4 Teilen Ether und 3 Teilen Petrolether verrührt. Das sich beim Stehen bildende Kristallisat wird abgesaugt. Man erhält 9,7 g (42 % der Theorie) des Produktes vom Schmelzpunkt 91 - 92°C.

Nach den vorausgehend beschriebenen Methoden werden die in der nachstehenden Tabelle aufgeführten Verbindungen der Formel (I) hergestellt:

Beispiel 8:

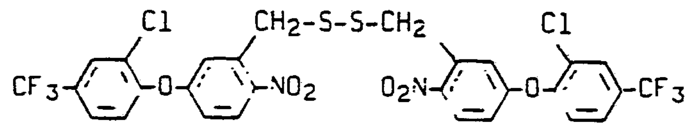

21,3 g (o,o4 mol) (2-Nitro-5-(2-chlor-4-trifluormethyl-phenoxy)-benzyl)-O-ethyl-dithiokohlensäureester wurden in 6o ml Ethanol gelöst, mit 2o ml einer konz. Ammoniumhydroxidlösung versetzt und 4 Tage bei Raumtemperatur stehen gelassen. Dann wird das Reaktionsgemisch auf Wasser gegossen und mit Toluol extrahiert. Die Toluolphase wird mit Wasser gewaschen und das Toluol in Vakuum abdestilliert. Man erhält 15 g (1oo% der Theorie) Bis-(2-nitro-5-(2-chlor-4-trifluormethylphenoxy)-benzyl)-disulfid vom Schmelzpunkt 1o4-1o7°C.

Beispiel 9:

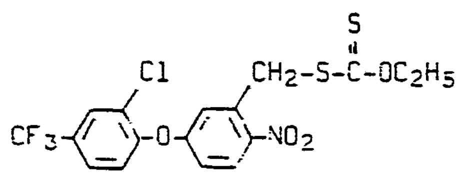

In eine Lösung von 8,4 g Kaliumxanthogenat in 5o ml Tetrahydrofuran werden bei -15°C 22,9 g 2-Nitro-5-(2-chlor-4-trifluormethyl-phenoxy)-benzyliodid, gelöst in 5o ml Tetrahydrofuran, getropft. Dann wird über Nacht bei 2o°C gerührt. Die Reaktionslösung wird dann auf Wasser gegossen, mit Toluol extrahiert, die Toluolphase wird mit Wasser gewaschen und dann das Toluol abdestilliert. Man erhält 17,7 g (78% der Theorie) S-(2-Nitro-5-(2-chlor-4-trifluormethyl-phenoxy)-benzyl)-O-ethyl-dithiokohlensäureester als gelbes Oel vom Brechungsindex $n_D^{20}$:1,5919.

Beispiel lo:

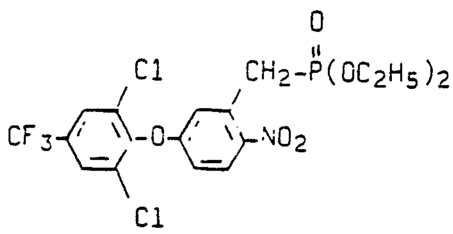

13 ml konz. Schwefelsäure und 13 ml konz. Salpetersäure werden bei einer Temperatur zwischen 0 und 5°C gemischt und auf -5°C gekühlt. Hierzu werden 22,8 g (o,o5 mol) 3-(2,6-Dichlor-4-trifluormethylphenoxy)-benzyl-phosphonsäure-diethylester, gelöst in 5o ml Methylenchlorid tropfenweise gegeben. Nach 3o Minuten wird das Reaktionsgemisch in Eiswasser gegossen, mit Methylenchlorid extrahiert, mit Wasser gewaschen, getrocknet und eingeengt. Der Rückstand wird mit Petrolether verrührt. Das kristalline Rohprodukt wird aus Isopropanol umkristallisiert. Man erhält 6.4 g (25% der Theorie) 2-Nitro-5-(2.6-dichlor-4-trifluormethyl-phenoxy)-benzyl-phosphonsäure-diethylester vom Schmelzpunkt 111°C.

Tabelle 2

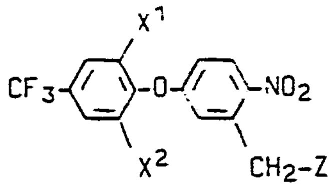

(I)

| Beispiel Nr. | $X^2$ | $X^1$ | Z | Brechungsindex bzw. Schmelzpunkt °C |
|---|---|---|---|---|
| 11 | H | Cl | $-O-CO-CH_2-Cl$ | 67 |
| 12 | H | Cl | $-S-CO-N(C_2H_5)_2$ | 1,5647 |
| 13 | H | Cl | $-O-CO-OC_2H_5$ | 1,5447 |
| 14 | Cl | Cl | $-O-CO-OC_2H_5$ | 83 |
| 15 | Cl | Cl | $-P(O)(OH)_2$ | 173 |
| 16 | H | Cl | $-S-CH_2-CO-OCH_3$ | $n_D^{20}$:1,5688 |
| 17 | H | Cl | $-S-CH_2-CO-OH$ | 93 |
| 18 | H | Cl | $-N(CH_3)_2$ | 1,5278 |
| 19 | H | Cl | -S- 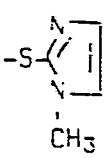 | 1o8 |

Tabelle 2 (Fortsetzung)

| Beispiel Nr. | $X^2$ | $X^1$ | Z | Brechungsindex bzw. Schmelzpunkt °C |
|---|---|---|---|---|
| 20 | H | Cl | 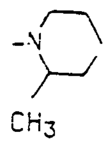 | $n_D^{20}$: 1,5389 |
| 21 | H | Cl | 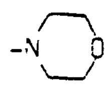 | 83 |
| 22 | H | Cl | 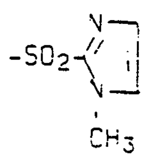 | 130 |
| 23 | H | Cl | 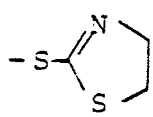 | 93 |
| 24 | H | Cl | $-S-CH_3$ | $n_D^{20}$ = 1,5849 |

Herstellung von Ausgangsstoffen

Beispiel II-1

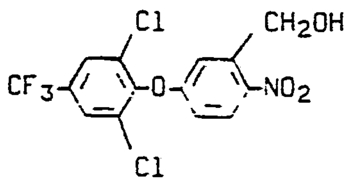

Eine Lösung von 41,5 g 5-(2,6-Dichlor-4-trifluormethyl-phenoxy)-2-nitro-benzoesäurechlorid in 100 ml Diglyme wird bei -5 bis -10°C zu einem Gemisch aus 4,2 g Natriumboranat und 60 ml Diglyme gegeben. Das Reaktionsgemisch wird 15 Stunden bei Raumtemperatur gerührt, dann mit 1,2 l Wasser verdünnt und mit Essigsäure angesäuert. Das ausfallende Produkt wird abgesaugt. Man erhält 36 g (94 % der Theorie) 5-(2,6-Dichlor-4-trifluormethyl-phenoxy)-2-nitro-benzylalkohol vom Schmelzpunkt 143°C.

Beispiel VI-1

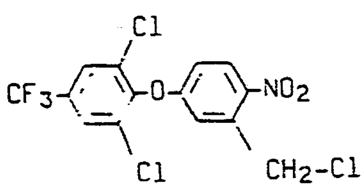

14,3 g (0,12 Mol) Thionylchlorid werden bei 20°C zu einer Mischung aus 38,2 g (0,1 Mol) 2-Nitro-5-(2,6-dichlor-4-trifluormethyl-phenoxy)-benzylalkohol, 80 ml Tetrahydrofuran und 1 ml Dimethylformamid gegeben. Das Reaktionsgemisch wird 150 Minuten unter Rückfluß zum

Sieden erhitzt, dann auf 1,3 l Wasser gegossen und abgesaugt. Man erhält 26,7 g (92 % der Theorie) 2-Nitro-5-(2,6-dichlor-4-trifluormethyl-phenoxy)-benzylchlorid in Form gelber Kristalle vom Schmelzpunkt 84°C.

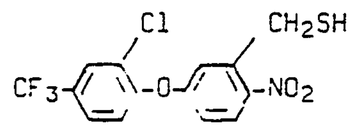

72,5 g (o,1 mol) Bis-(2-nitro-5-(2-chlor-4-trifluormethyl-phenoxy)-benzyl)-disulfid werden in 5o ml siedendem Alkohol gelöst. Dann stellt man sich eine Natriumdisulfidlösung aus 17,5 g Natriumsulfid-nonahydrat und 2,3 g Schwefel her und tropft diese Lösung innerhalb von 2o Min. zu der Disulfidlösung. Unter Rückfluß wird dann eine alkoholische Lösung von 4 g Natriumhydroxid in 2o Min. zugetropft. Dann wird auf ein Gemisch aus 1oo g Eis und 15o g Wasser gegossen. Dann wird mit Salzsäure angesäuert. Die ausgeschiedene Mercaptoverbindung wird dann mit Toluol extrahiert. Das Toluol wird abdestilliert. Man erhält 36 g (5o% der Theorie) 2-Nitro-5-(2-chlor-4-trifluormethyl-phenoxy)-benzylmercaptan.

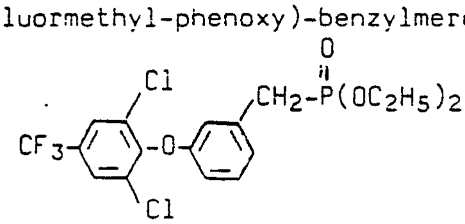

Eine Mischung aus 35,6 g (o,1 mol) 3-(2,6-Dichlor-4-trifluormethyl-phenoxy)-benzylchlorid und 5o ml Triethylphosphit wird 13 Stunden bei 156°C am Wasserabscheider erhitzt. Nach Einengen wird der Rückstand mit Diethylether verrührt, wobei man nach Absaugen 32,3 g (71% der Theorie) 3-(2,6-Dichlor-4-trifluormethyl-phenoxy)-benzyl-phosphonsäure-diethylester vom Schmelzpunkt 94,5°C erhält.

Analog erhält man in 96%iger Ausbeute 3-(2-Chlor-4-trifluormethyl-phenoxy)-benzyl-phosphonsäure-diethylester vom Schmelzpunkt 39°C; ebenso in 81%iger Ausbeute die 3-(2-Chlor-4-trifluormethyl-phenoxy)-benzyl-phosphonsäure vom Schmelzpunkt 184°C.

In den nachfolgenden Verwendungsbeispielen wird die nachstehend angegebene Verbindung als Vergleichssubstanz eingesetzt:

(A) =

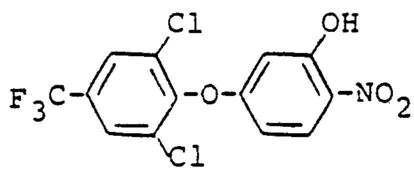

2-Nitro-5-(2,6-dichlor-4-trifluormethyl-phenoxy)-phenol
(bekannt aus US-PS 3 928 416)

Beispiel A

Entlaubung und Austrocknung der Blätter bei Baumwolle

Lösungsmittel: 30 Gewichtsteile Dimethylformamid
Emulgator: 1 Gewichtsteil Polyoxyethylen-Sorbitan-Monolaurat

Zur Herstellung einer zweckmäßigen Wirkstoffzubereitung vermischt man 1 Gewichtsteil Wirkstoff mit den angegebenen Mengen Lösungsmittel und Emulgator und füllt mit Wasser auf die gewünschte Konzentration auf.

Baumwollpflanzen werden im Gewächshaus bis zur vollen Entfaltung des 5. Folgeblattes angezogen. In diesem Stadium werden die Pflanzen tropfnaß mit den Wirkstoffzubereitungen besprüht. Nach 1 Woche werden der Blattfall und das Austrocknen der Blätter im Vergleich zu den Kontrollpflanzen bonitiert. Es bedeuten:

0 kein Austrocknen der Blätter, kein Blattfall
+ leichtes Austrocknen der Blätter, geringer Blattfall
++ starkes Austrocknen der Blätter, starker Blattfall
+++ sehr starkes Austrocknen der Blätter, sehr starker Blattfall

In diesem Test zeigt die erfindungsgemäße Verbindung (1) eine starke Wirksamkeit.

Patentansprüche

1) Substituierte 2-Nitro-5-(4-trifluormethyl-phenoxy)-toluole der Formel

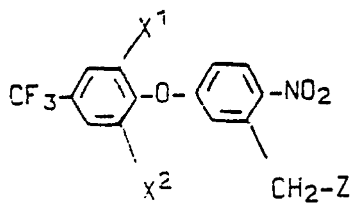

(I)

in welcher

$X^1$ und $X^2$ unabhängig voneinander für Wasserstoff oder Halogen stehen und

Z für Cyano oder für einen gegebenenfalls durch Halogen substituierten Rest aus der Reihe Alkenoxy, Alkinoxy, Benzyloxy, Alkylcarbonyloxy, Alkoxycarbonyloxy, Alkylthio, Alkenylthio, Alkinylthio, Benzylthio, Alkylsulfinyl, Alkenylsulfinyl, Alkinylsulfinyl, Benzylsulfinyl, Alkylsulfonyl, Alkenylsulfonyl, Alkinylsulfonyl, Benzylsulfonyl, Hydroxycarbonylalkylthio, Alkoxycarbonylalkylthio, Alkoxythiocarbonylthio, Dialkylaminocarbonylthio, Alkoxycarbonylalkylsulfinyl, Alkoxycarbonylalkylsulfonyl, Hydroxycarbonylalkylsulfinyl und Hydroxycarbonylalkylsulfonyl, oder für einen gegebenenfalls durch Halogen, Cyano, Nitro, Alkyl, Trifluormethyl, Alkoxy, Alkylthio, Alkylsulfinyl und/oder Alkylsulfonyl substituierten Rest aus der Reihe Phenoxy, Phenylthio, Phenylsulfinyl, Phenylsulfonyl, Pyridinoxy oder Pyridinthio steht,

oder

Z für den Rest der Formel

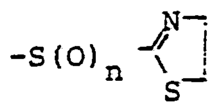 oder

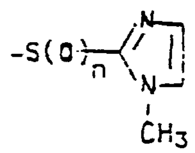 steht, worin

n jeweils für die Zahlen 0, 1 oder 2 steht,

oder

Z für den Rest der Formel -S-S-$CH_2$

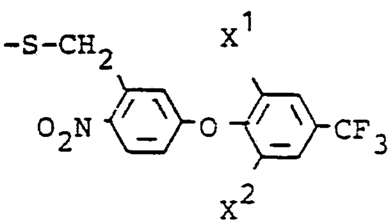

steht, worin

$X^1$ und $X^2$ die oben angegebene Bedeutung haben,

oder

Z für den Rest der Formel 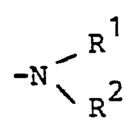 steht, worin

$R^1$ für Wasserstoff, für gegebenenfalls durch Halogen, Cyano oder Alkoxy substituiertes Alkyl, für Alkenyl, Alkinyl, Cycloalkyl oder Benzyl steht und

$R^2$ für Wasserstoff, für gegebenenfalls durch Halogen, Cyano, Alkoxy, Hydroxycarbonyl oder Alkoxycarbonyl substituiertes Alkyl, für Alkenyl, Alkinyl, Cycloalkyl oder Benzyl steht, oder für Naphthyl oder Phenyl steht, wobei jeder dieser beiden Reste gegebenenfalls durch Halogen, Cyano, Nitro, Alkyl, Trifluormethyl, Alkoxy, Alkylthio, Alkylsulfinyl und/oder Alkylsulfonyl substituiert ist,

oder

$R^1$ und $R^2$ zusammen mit dem Stickstoffatom, an das sie gebunden sind, einen gegebenenfalls durch Halogen, Alkyl, Halogenalkyl, Alkoxy, Alkylthio, Cyano, Alkylcarbonyl, Alkoxycarbonyl und/oder Phenyl substituierten gesättigten oder ungesättigten und gegebenenfalls benzanellierten Mono- oder Bicyclus bilden, der gegebenenfalls Sauerstoff, Schwefel und/oder Stickstoff als weitere Heteroatome enthält,

oder

Z für den Rest der Formel 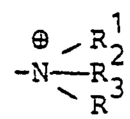 $X^{\ominus}$ steht, worin

$R^1$ und $R^2$ die oben angegebenen Bedeutungen haben,

$R^3$ für Wasserstoff oder Alkyl steht und

X für Halogen steht,

wobei, - für den Fall, daß $R^1$ und $R^2$ zusammen mit dem Stickstoffatom, an das sie gebunden sind, einen Ring bilden, welcher wenigstens ein weiteres Stickstoffatom enthält - , der Rest $R^3$ auch an ein anderes Stickstoffatom gebunden sein kann als an dasjenige, das die Bindung zum übrigen Molekülteil ausübt,

oder

Z auch für den Rest der Formel 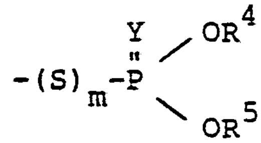 steht, worin

m für die Zahlen 0 oder 1 steht,

Y für Sauerstoff und, - für den Fall, daß m für 1 steht - , auch für Schwefel steht,

$R^4$ für Wasserstoff, Natrium, Kalium oder Alkyl steht und

$R^5$ für Wasserstoff, Natrium, Kalium oder Alalkyl steht, oder zusammen mit $R^4$ für Calcium steht.

2) Substituierte 2-Nitro-5-(4-trifluormethyl-phenoxy)-toluole der Formel (I), in denen

$X^1$ für Chlor steht,

$X^2$ für Wasserstoff oder Chlor steht und

Z für Cyano oder für einen gegebenenfalls durch Fluor und/oder Chlor substituierten Rest aus der Reihe $C_3$-$C_5$-Alkenoxy, $C_3$-$C_5$-Alkinoxy, Benzyloxy, $C_1$-$C_4$-Alkyl-carbonyloxy, $C_1$-$C_4$-Alkoxycarbonyloxy, $C_1$-$C_4$-Alkylthio, $C_3$-$C_5$-Alkenylthio, $C_3$-$C_5$-Alkinylthio, Benzylthio, $C_1$-$C_4$-Alkylsulfinyl, $C_3$-$C_5$-Alkenylsulfinyl, $C_3$-$C_5$-Alkinylsulfinyl, Benzylsulfinyl, $C_1$-$C_4$-Alkylsulfonyl, $C_3$-$C_5$-Alkenylsulfonyl, $C_3$-$C_5$-Alkinylsulfonyl, Benzylsulfonyl, Hydroxycarbonyl-$C_1$-$C_2$-alkylthio, $C_1$-$C_4$-Alkoxycarbonyl-$C_1$-$C_2$-alkylthio, $C_1$-$C_4$-Alkoxythiocarbonylthio oder Di-($C_1$-$C_4$-alkyl)-aminocarbonylthio steht,
oder für einen gegebenenfalls durch Fluor, Chlor, Brom, Cyano, Nitro, $C_1$-$C_4$-Alkyl, Trifluormethyl, $C_1$-$C_4$-Alkoxy, $C_1$-$C_4$-Alkylthio, $C_1$-$C_4$-Alkylsulfinyl und/oder $C_1$-$C_4$-Alkylsulfonyl substituierten Rest aus der Reihe Phenoxy, Phenylthio, Phenylsulfinyl, Phenylsulfonyl, Pyridinoxy oder Pyridinthio steht,

oder

Z für den Rest der Formel $-S(O)_n-$ 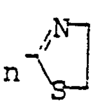 oder

$-S(O)_n-$ 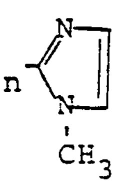 steht, worin

n jeweils für die Zahlen 0, 1 oder 2 steht,

oder

Z für den Rest der Formel

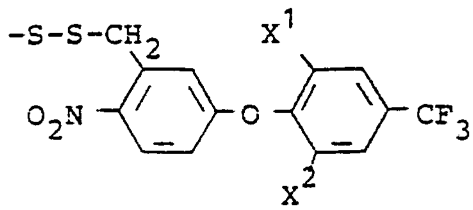

steht, worin

$X^1$ und $X^2$ die oben angegebene Bedeutung haben,

oder

Z für den Rest der Formel

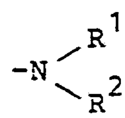

steht, worin

$R^1$ für Wasserstoff, für gegebenenfalls durch Fluor, Chlor, Cyano oder $C_1$-$C_4$-Alkoxy substituiertes $C_1$-$C_4$-Alkyl, für $C_3$-$C_5$-Alkenyl, $C_3$-$C_5$-Alkinyl, $C_5$-$C_6$-Cycloalkyl oder Benzyl steht und

$R^2$ für Wasserstoff, für gegebenenfalls durch Fluor, Chlor, Cyano, $C_1$-$C_4$-Alkoxy, Hydroxycarbonyl oder $C_1$-$C_4$-Alkoxy-carbonyl substituiertes $C_1$-$C_4$-Alkyl, für $C_3$-$C_5$-Alkenyl,

$C_3$-$C_5$-Alkinyl, $C_5$-$C_6$-Cycloalkyl oder Benzyl steht, oder für Naphthyl oder Phenyl steht, wobei jeder der beiden letztgenannten Reste gegebenenfalls durch Fluor, Chlor, Brom, Cyano, Nitro, $C_1$-$C_4$-Alkyl, Trifluormethyl, $C_1$-$C_4$-Alkoxy, $C_1$-$C_4$-Alkylthio, $C_1$-$C_4$-Alkylsulfinyl und/oder $C_1$-$C_4$-Alkylsulfonyl substituiert ist,

oder

$R^1$ und $R^2$ zusammen mit dem Stickstoffatom, an das sie gebunden sind, einen gegebenenfalls durch Fluor, Chlor, Brom, Iod, Cyano, $C_1$-$C_4$-Alkyl, Trifluormethyl, $C_1$-$C_4$-Alkoxy, $C_1$-$C_4$-Alkylthio, $C_1$-$C_4$-Alkylcarbonyl, $C_1$-$C_4$-Alkoxy-carbonyl und/oder Phenyl substituierten gesättigten oder ungesättigten und gegebenenfalls benzannellierten Mono- oder Bicyclus bilden, welcher 3 bis 12 Kohlenstoffatome und gegebenenfalls Sauerstoff, Schwefel und/oder Stickstoff als weitere Heteroatome enthält,

oder

Z für den Rest der Formel 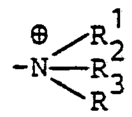 $X^{\ominus}$ steht, worin

$R^1$ und $R^2$ die oben angegebenen Bedeutungen haben,

$R^3$ für Wasserstoff oder $C_1$-$C_4$-Alkyl steht und

X für Chlor, Brom oder Iod steht,

wobei, - für den Fall, daß $R^1$ und $R^2$ zusammen mit dem Stickstoffatom, an das sie gebunden sind, einen Ring bilden, welcher wenigstens ein weiteres Stickstoffatom enthält - , der Rest $R^3$ auch an ein anderen Stickstoffatom gebunden sein kann als an dasjenige, das die Bindung zum übrigen Molekülteil ausübt,

oder

Z auch für den Rest der Formel 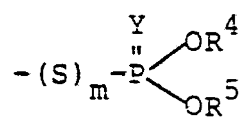

steht, worin

m für die Zahlen 0 oder 1 steht,

Y für Sauerstoff und, - für den Fall, daß m für 1 steht - , auch für Schwefel steht,

$R^4$ für Wasserstoff, Natrium, Kalium oder $C_1$-$C_4$-Alkyl steht und

$R^5$ für Wasserstoff, Natrium, Kalium oder $C_1$-$C_4$-Alkyl steht oder zusammen mit $R^4$ für Calcium steht.

3) Verfahren zur Herstellung von substituierten 2-Nitro-5-(4-trifluormethyl-phenoxy)-toluolen der Formel

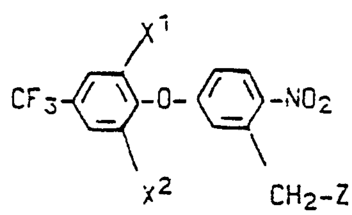

(I)

in welcher

$X^1$ und $X^2$ unabhängig voneinander für Wasserstoff oder Halogen stehen und

Z für Cyano oder für einen gegebenenfalls durch Halogen substituierten Rest aus der Reihe Alkenoxy, Alkinoxy, Benzyloxy, Alkylcarbonyloxy, Alkoxycarbonyloxy, Alkylthio, Alkenylthio, Alkinylthio, Benzylthio, Alkylsulfinyl, Alkenylsulfinyl, Alkinylsulfinyl, Benzylsulfinyl, Alkylsulfonyl, Alkenylsulfonyl, Alkinylsulfonyl, Benzylsulfonyl, Hydroxycarbonylalkylthio, Alkoxycarbonylalkylthio, Alkoxythiocarbonylthio, Dialkylaminocarbonylthio, Alkoxycarbonylalkylsulfinyl, Alkoxycarbonylalkylsulfonyl, Hydroxycarbonylalkylsulfinyl und Hydroxycarbonylalkylsulfonyl, oder

für einen gegebenenfalls durch Halogen, Cyano, Nitro, Alkyl, Trifluormethyl, Alkoxy, Alkylthio, Alkylsulfinyl und/oder Alkylsulfonyl substituierten Rest aus der Reihe Phenoxy, Phenylthio, Phenylsulfinyl, Phenylsulfonyl, Pyridinoxy oder Pyridinthio steht,

oder

Z für den Rest der Formel

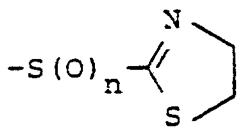 oder 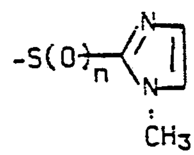 steht, worin

n jeweils für die Zahlen 0, 1 oder 2 steht,

oder

Z für den Rest der Formel

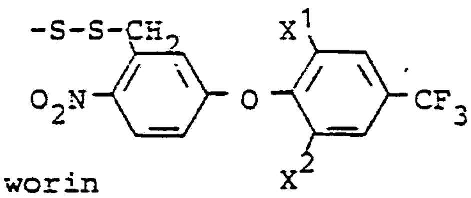

steht, worin

$X^1$ und $X^2$ die oben angegebene Bedeutung haben,

oder

Z für den Rest der Formel 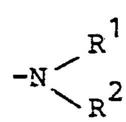 steht, worin

$R^1$ für Wasserstoff, für gegebenenfalls durch Halogen, Cyano oder Alkoxy substituiertes Alkyl, für Alkenyl, Alkinyl, Cycloalkyl oder Benzyl steht und

$R^2$ für Wasserstoff, für gegebenenfalls durch Halogen, Cyano, Alkoxy, Hydroxycarbonyl oder Alkoxycarbonyl substituiertes Alkyl, für Alkenyl, Alkinyl, Cycloalkyl oder Benzyl steht, oder für Naphthyl oder Phenyl steht, wobei jeder dieser beiden Reste gegebenenfalls durch Halogen, Cyano, Nitro, Alkyl, Trifluormethyl, Alkoxy, Alkylthio, Alkylsulfinyl und/oder Alkylsulfonyl substituiert ist,

oder

$R^1$ und $R^2$ zusammen mit dem Stickstoffatom, an das sie gebunden sind, einen gegebenenfalls durch Halogen, Alkyl, Halogenalkyl, Alkoxy, Alkylthio, Cyano, Alkylcarbonyl, Alkoxycarbonyl und/oder Phenyl substituierten gesättigten oder ungesättigten und gegebenenfalls benzanellierten Mono- oder Bicyclus bilden, der gegebenenfalls Sauerstoff, Schwefel und/oder Stickstoff als weitere Heteroatome enthält,

oder

Z für den Rest der Formel 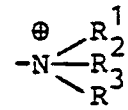 $X^{\ominus}$ steht, worin

$R^1$ und $R^2$ die oben angegebenen Bedeutungen haben,

$R^3$ für Wasserstoff oder Alkyl steht und

X für Halogen steht,

wobei, - für den Fall, daß $R^1$ und $R^2$ zusammen mit dem Stickstoffatom, an das sie gebunden sind, einen Ring bilden, welcher wenigstens ein weiteres Stickstoffatom enthält - , der Rest $R^3$ auch an ein anderes Stickstoffatom gebunden sein kann als an dasjenige, das die Bindung zum übrigen Molekülteil ausübt,

oder

Z auch für den Rest der Formel 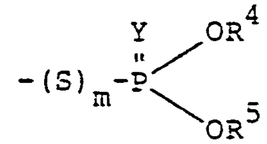 steht, worin

m für die Zahlen 0 oder 1 steht,

Y für Sauerstoff und, - für den Fall, daß m für 1 steht - , auch für Schwefel steht,

$R^4$ für Wasserstoff, Natrium, Kalium oder Alkyl steht und

$R^5$ für Wasserstoff, Natrium, Kalium oder Alalkyl steht, oder zusammen mit $R^4$ für Calcium steht,

dadurch gekennzeichnet, daß man

(a) 2-Nitro-5-(4-trifluormethyl-phenoxy)-benzylalkohole der Formel

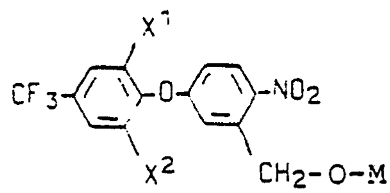

(II)

in welcher

$X^1$ und $X^2$ die oben angegebene Bedeutung haben und

M für Wasserstoff, Natrium oder Kalium steht,

mit Halogenverbindungen der Formel

$$Hal\text{-}Z^1 \quad (III)$$

in welcher

Hal für Chlor, Brom oder Iod steht und

$Z^1$ für einen gegebenenfalls durch Halogen substituierten Rest aus der Reihe Alkenyl, Alkinyl, Benzyl, Alkylcarbonyl oder Alkoxycarbonyl steht,

gegebenenfalls in Gegenwart eines Säureakzeptors und gegebenenfalls in Gegenwart eines Verdünnungsmittels umsetzt,

oder

(b) 2-Nitro-5-(4-trifluormethyl-phenoxy)-benzyl-thiole der Formel

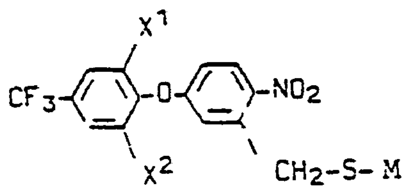

(IV)

in welcher

$X^1$, $X^2$ und M die oben angegebene Bedeutung haben,

mit Halogenverbindungen der Formel

$$Hal\text{-}Z^2 \quad (V)$$

in welcher

Hal die oben angegebene Bedeutung hat und

$Z^2$ für einen gegebenenfalls durch Halogen substituierten Rest aus der Reihe Alkenyl, Alkinyl, Benzyl, Alkyl oder Dialkylaminocarbonyl steht,

gegebenenfalls in Gegenwart eines Säureakzeptors und gegebenenfalls in Gegenwart eines Verdünnungsmittels umsetzt,

oder

(c) 2-Nitro-5-(4-trifluormethyl-phenoxy)-benzylthioverbindungen der Formel

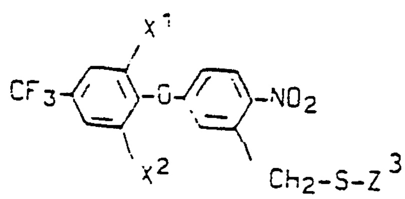

(Ia)

in welcher

$X^1$ und $X^2$ die oben angegebene Bedeutung haben und

$Z^3$ für Wasserstoff oder einen gegebenenfalls durch Halogen substituierten Rest aus der Reihe Alkyl, Alkenyl, Alkinyl, Benzyl, Hydroxycarbonylalkyl oder Alkoxycarbonylalkyl steht, oder für gegebenenfalls durch Halogen, Cyano, Nitro, Alkyl, Trifluormethyl, Alkoxy, Alkylthio, Alkylsulfinyl und/oder Alkylsulfonyl substituiertes Phenyl steht oder für den Rest der Formel

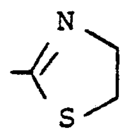 oder 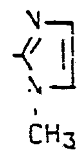 steht,

mit Oxidationsmitteln gegebenenfalls in Gegenwart eines Verdünnungsmittels umsetzt,

oder

(d) 2-Nitro-5-(4-trifluormethyl-phenoxy)-benzylhalogenide der Formel

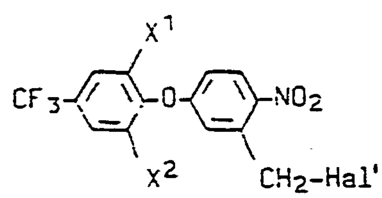

(VI)

in welcher

$X^1$ und $X^2$ die oben angegebene Bedeutung haben und

Hal' für Chlor, Brom oder Iod steht,

α) mit Nucleophilen der Formel

$$M^1\text{-}Z \qquad (VII)$$

in welcher

Z die oben angegebene Bedeutung hat und

$M^1$ für Wasserstoff, Natrium, Kalium oder Ammonium steht,

gegebenenfalls in Gegenwart eines Säureakzeptors sowie gegebenenfalls in Gegenwart eines Verdünnungsmittels umsetzt,

oder

ß) mit Trialkylphosphiten der Formel

$$P(OR)_3 \qquad (VIII)$$

in welcher

R für Alkyl steht,

gegebenenfalls in Gegenwart eines Katalysators und gegebenenfalls in Gegenwart eines Verdünnungsmittels umsetzt und gegebenenfalls anschließend die dabei entstehenden Phosphonsäureester der Formel

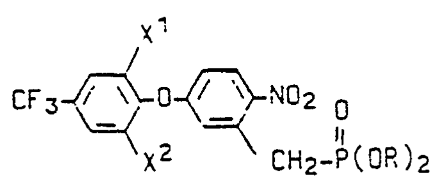

(Ib)

in welcher

$X^1$, $X^2$ und R die oben angegebene Bedeutung haben,

gegebenenfalls in Gegenwart eines Verdünnungsmittels verseift und gegebenenfalls die dabei entstehenden Säuren der Formel

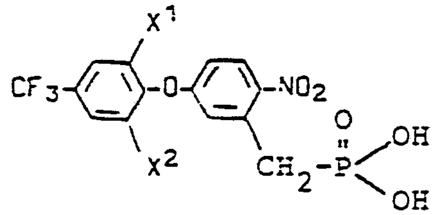

(Ic)

in welcher

$X^1$ und $X^2$ die oben angegebene Bedeutung haben,

mit einer Metallverbindung der Formel

$$M^2-X^3 \qquad (IX)$$

in welcher

$M^2$ für Natrium, Kalium oder ein Calcium-äquivalent steht und

$X^3$ für Chlor oder Hydroxy steht,

gegebenenfalls in Gegenwart eines Verdünnungsmittels umsetzt,

oder

(e) 2-Nitro-5-(4-trifluormethyl-phenoxy)-benzylamine der Formel

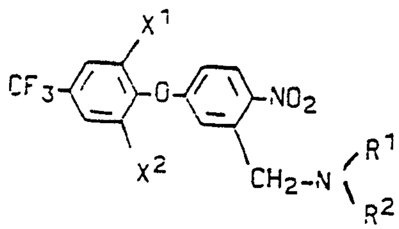

(Id)

in welcher

$X^1$, $X^2$, $R^1$ und $R^2$ die oben angegebene Bedeutung haben,

mit Halogeniden der Formel

Hal"-$R^3$ (X)

in welcher

$R^3$ die oben angegebene Bedeutung hat und

Hal" für Chlor, Brom oder Iod steht,

gegebenenfalls in Gegenwart eines Verdünnungsmittels umsetzt,

oder

(f) substituierte 2-Nitro-5-(4-trifluormethyl-phenoxy)-toluole der Formel

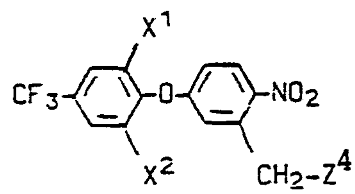

(Ie)

in welcher

$X^1$ und $X^2$ die oben angegebene Bedeutung haben und

$Z^4$ für Alkoxycarbonylalkylthio oder Alkoxycarbonylalkylamino steht,

mit Alkalihydroxiden in Gegenwart eines Verdünnungsmittels umsetzt und die dabei entstehenden Carbonsäuresalze mit Mineralsäuren behandelt,

oder

(g) 3-(4-Trifluormethyl-phenoxy)-toluole der Formel

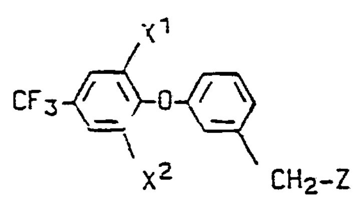

(XI)

in welcher

$X^1$, $X^2$ und Z die oben angegebene Bedeutung haben,

mit Salpetersäure gegebenenfalls in Gegenwart eines Katalysators und gegebenenfalls in Gegenwart eines Verdünnungsmittels umsetzt.

4) Herbizide und pflanzenwuchsregulierende Mittel, gekennzeichnet durch einen Gehalt an mindestens einem substituierten 2-Nitro-5-(4-trifluormethyl-phenoxy)-toluol der Formel (I).

5) Verwendung von substituierten 2-Nitro-5-(4-trifluormethyl-phenoxy)-toluolen der Formel (I) zur Bekämpfung von Unkräutern sowie zur Regulierung des Pflanzenwachstums.

6) Verfahren zur Herstellung von herbiziden und pflanzenwuchsregulierenden Mitteln, dadurch gekennzeichnet, daß man substituierte 2-Nitro-5-(4-trifluormethyl-phenoxy)-toluole der Formel (I) mit Streckmitteln und/oder oberflächenaktiven Stoffen vermischt.

7) 2-Nitro-5-(4-trifluormethyl-phenoxy)-benzylthiole der Formel

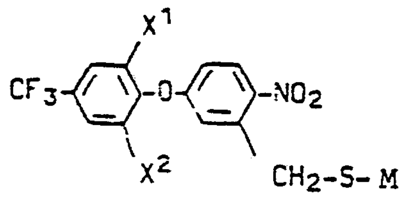

(IV)

in welcher

$X^1$ und $X^2$ unabhängig voneinander für Wasserstoff oder Halogen stehen und

M für Wasserstoff, Natrium oder Kalium steht.

8) Verfahren zur Herstellung von 2-Nitro-5-(4-trifluormethyl-phenoxy)-benzylthiolen der Formel

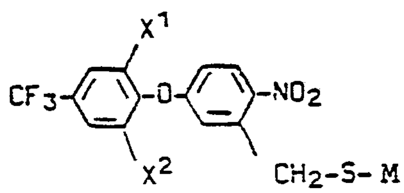

(IV)

in welcher

$X^1$ und $X^2$ unabhängig voneinander für Wasserstoff oder Halogen stehen und

M für Wasserstoff, Natrium oder Kalium steht,

dadurch gekennzeichnet, daß man Bis-{2-Nitro-5-(4-trifluormethyl-phenoxy)-benzyl)-disulfide der Formel

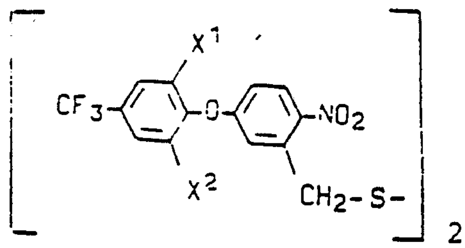

(If)

in welcher

$X^1$ und $X^2$ die oben angegebene Bedeutung haben,

mit Natriumsulfid und/oder Natriumdisulfid und Natriumhydroxid in Gegenwart von Wasser und gegebenenfalls in Gegenwart eines organischen Lösungsmittels umsetzt und gegebenenfalls ansäuert oder mit Kaliumhydroxid behandelt.

9) 3-(4-Trifluormethyl-phenoxy)-toluole der Formel

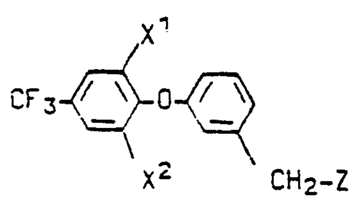

(XI)

in welcher

$X^1$ und $X^2$ unabhängig voneinander für Wasserstoff oder Halogen stehen und

Z für Cyano oder für einen gegebenenfalls durch Halogen substituierten Rest aus der Reihe Alkenoxy, Alkinoxy, Benzyloxy, Alkylcarbonyloxy, Alkoxycarbonyloxy, Alkylthio, Alkenylthio, Alkinylthio, Benzylthio, Alkylsulfinyl, Alkenylsulfinyl, Alkinylsulfinyl, Benzylsulfinyl, Alkylsulfonyl, Alkenylsulfonyl, Alkinylsulfonyl, Benzylsulfonyl, Hydroxycarbonylalkylthio, Alkoxycarbonylalkylthio, Alkoxythiocarbonylthio, Dialkylaminocarbonylthio, Alkoxycarbonylalkylsulfinyl, Alkoxycarbonylalkylsulfonyl, Hydroxycarbonylalkylsulfinyl und Hydroxycarbonylalkylsulfonyl, oder für einen gegebenenfalls durch Halogen, Cyano, Nitro, Alkyl, Trifluormethyl, Alkoxy, Alkylthio, Alkylsulfinyl und/oder Alkylsulfonyl substituierten Rest aus der Reihe Phenoxy, Phenylthio, Phenylsulfinyl, Phenylsulfonyl, Pyridinoxy oder Pyridinthio steht,

oder

Z für den Rest der Formel 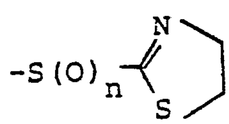 oder 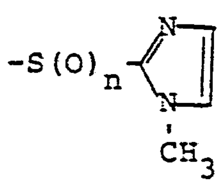 steht, worin

n jeweils für die Zahlen 0, 1 oder 2 steht,

oder

Z für den Rest der Formel

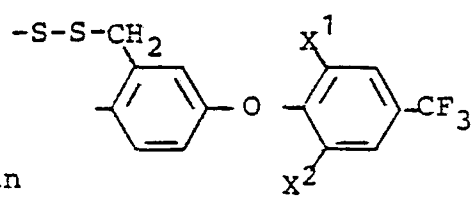

steht, worin

$X^1$ und $X^2$ die oben angegebene Bedeutung haben,

Z für den Rest der Formel 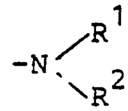 steht, worin

$R^1$ für Wasserstoff, für gegebenenfalls durch Halogen, Cyano oder Alkoxy substituiertes Alkyl, für Alkenyl, Alkinyl, Cycloalkyl oder Benzyl steht und

$R^2$ für Wasserstoff, für gegebenenfalls durch Halogen, Cyano, Alkoxy, Hydroxycarbonyl oder Alkoxycarbonyl substituiertes Alkyl, für Alkenyl, Alkinyl, Cycloalkyl oder Benzyl steht, oder für Naphthyl oder Phenyl steht, wobei jeder dieser beiden Reste gegebenenfalls durch Halogen, Cyano, Nitro, Alkyl, Trifluormethyl, Alkoxy, Alkylthio, Alkylsulfinyl und/oder Alkylsulfonyl substituiert ist,

oder

$R^1$ und $R^2$ zusammen mit dem Stickstoffatom, an das sie gebunden sind, einen gegebenenfalls durch Halogen, Alkyl, Halogenalkyl, Alkoxy, Alkylthio, Cyano, Alkylcarbonyl, Alkoxycarbonyl und/oder Phenyl substituierten gesättigten oder ungesättigten und gegebenenfalls benzanellierten Mono- oder Bicyclus bilden, der gegebenenfalls Sauerstoff, Schwefel und/oder Stickstoff als weitere Heteroatome enthält,

oder

Z für den Rest der Formel 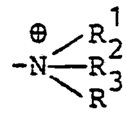 $X^{\ominus}$ steht, worin

$R^1$ und $R^2$ die oben angegebenen Bedeutungen haben,

$R^3$ für Wasserstoff oder Alkyl steht und

X für Halogen steht,

wobei, - für den Fall, daß $R^1$ und $R^2$ zusammen mit dem Stickstoffatom, an das sie gebunden sind, einen Ring bilden, welcher wenigstens ein weiteres Stickstoffatom enthält - , der Rest $R^3$ auch an ein anderes Stickstoffatom gebunden sein kann als an dasjenige, das die Bindung zum übrigen Molekülteil ausübt,

oder

Z auch für den Rest der Formel 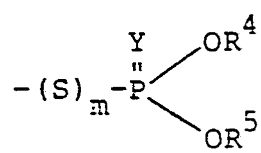 steht, worin

m für die Zahlen 0 oder 1 steht,

Y für Sauerstoff und, - für den Fall, daß m für 1 steht - , auch für Schwefel steht,

$R^4$ für Wasserstoff, Natrium, Kalium oder Alkyl steht und

$R^5$ für Wasserstoff, Natrium, Kalium oder Alkyl steht, oder zusammen mit $R^4$ für Calcium steht.

10) Verfahren zur Herstellung von 3-(4-Trifluormethyl-phenoxy)-toluolen der Formel

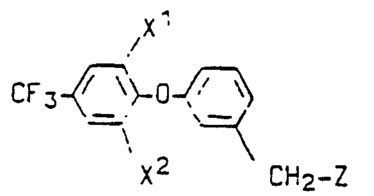

(XI)

in welcher

$X^1$ und $X^2$ unabhängig voneinander für Wasserstoff oder Halogen stehen und

Z für Cyano oder für einen gegebenenfalls durch Halogen substituierten Rest aus der Reihe Alkenoxy, Alkinoxy, Benzyloxy, Alkylcarbonyloxy, Alkoxycarbonyloxy, Alkylthio, Alkenylthio, Alkinylthio, Benzylthio, Alkylsulfinyl, Alkenylsulfinyl, Alkinylsulfinyl, Benzylsulfinyl, Alkylsulfonyl, Alkenylsulfonyl, Alkinylsulfonyl, Benzylsulfonyl, Hydroxycarbonylalkylthio, Alkoxycarbonylalkylthio, Alkoxythiocarbonylthio, Dialkylaminocarbonylthio, Alkoxycarbonylalkylsulfinyl, Alkoxycarbonylalkylsulfonyl, Hydroxycarbonylalkylsulfinyl und Hydroxycarbonylalkylsulfonyl, oder für einen gegebenenfalls durch Halogen, Cyano, Nitro, Alkyl, Trifluormethyl, Alkoxy, Alkylthio, Alkylsulfinyl und/oder Alkylsulfonyl substituierten Rest aus der Reihe Phenoxy, Phenylthio, Phenylsulfinyl, Phenylsulfonyl, Pyridinoxy oder Pyridinthio steht,

oder

Z für den Rest der Formel 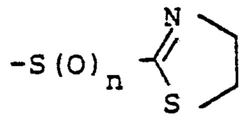 oder 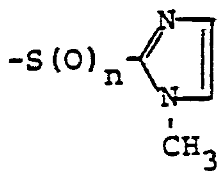 steht, worin

n jeweils für die Zahlen 0, 1 oder 2 steht,

oder

Z für den Rest der Formel

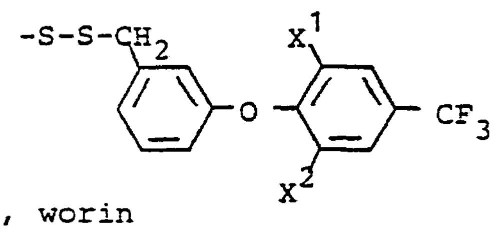

steht, worin

$X^1$ und $X^2$ die oben angegebene Bedeutung haben,

oder

Z für den Rest der Formel 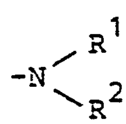 steht, worin

$R^1$ für Wasserstoff, für gegebenenfalls durch Halogen, Cyano oder Alkoxy substituiertes Alkyl, für Alkenyl, Alkinyl, Cycloalkyl oder Benzyl steht und

$R^2$ für Wasserstoff, für gegebenenfalls durch Halogen, Cyano, Alkoxy, Hydroxycarbonyl oder Alkoxycarbonyl substituiertes Alkyl, für Alkenyl, Alkinyl, Cycloalkyl oder Benzyl steht, oder für Naphthyl oder Phenyl steht, wobei jeder dieser beiden Reste gegebenenfalls durch Halogen, Cyano, Nitro, Alkyl, Trifluormethyl, Alkoxy, Alkylthio, Alkylsulfinyl und/oder Alkylsulfonyl substituiert ist,

oder

$R^1$ und $R^2$ zusammen mit dem Stickstoffatom, an das sie gebunden sind, einen gegebenenfalls durch Halogen, Alkyl, Halogenalkyl, Alkoxy, Alkylthio, Cyano, Alkylcarbonyl, Alkoxycarbonyl und/oder Phenyl substituierten gesättigten oder ungesättigten und gegebenenfalls benzanellierten Mono- oder Bicyclus bilden, der gegebenenfalls Sauerstoff, Schwefel und/oder Stickstoff als weitere Heteroatome enthält,

oder

Z für den Rest der Formel 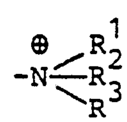 $X^{\ominus}$ steht, worin

$R^1$ und $R^2$ die oben angegebenen Bedeutungen haben,

$R^3$ für Wasserstoff oder Alkyl steht und

X für Halogen steht,

wobei, - für den Fall, daß $R^1$ und $R^2$ zusammen mit dem Stickstoffatom, an das sie gebunden sind, einen Ring bilden, welcher wenigstens ein weiteres Stickstoffatom enthält - , der Rest $R^3$ auch an ein anderes Stickstoffatom gebunden sein kann als an dasjenige, das die Bindung zum übrigen Molekülteil ausübt,

oder

Z auch für den Rest der Formel 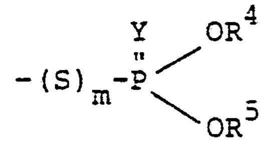 steht, worin

m für die Zahlen 0 oder 1 steht,

Y für Sauerstoff und, - für den Fall, daß m für 1 steht - , auch für Schwefel steht,

$R^4$ für Wasserstoff, Natrium, Kalium oder Alkyl steht und

$R^5$ für Wasserstoff, Natrium, Kalium oder Alalkyl steht, oder zusammen mit $R^4$ für Calcium steht,

dadurch gekennzeichnet, daß man

(h) 3-(4-Trifluormethyl-phenoxy)-benzylalkohole der Formel

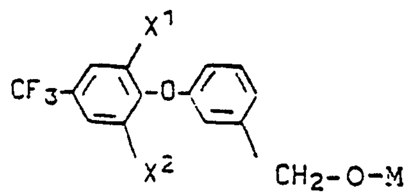

(XIV)

in welcher

$X^1$, $X^2$ und M die oben angegebene Bedeutung haben,

mit Halogenverbindungen der Formel

$$Hal-Z^1 \qquad (III)$$

in welcher

Hal und $Z^1$ die oben angegebene Bedeutung haben,

gegebenenfalls in Gegenwart eines Säureakzeptors und gegebenenfalls in Gegenwart eines Verdünnungsmittels umsetzt,

oder

(i) 3-(4-Trifluormethyl-phenoxy)-benzylthiole der Formel

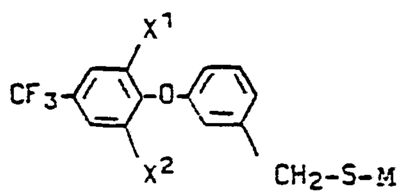

(XV)

in welcher

$X^1$, $X^2$ und M die oben angegebene Bedeutung haben,

mit Halogenverbindungen der Formel

$$Hal-Z^2 \qquad (V)$$

in welcher

Hal und $Z^2$ die oben angegebene Bedeutung haben,

gegebenenfalls in Gegenwart eines Säureakzeptors und gegebenenfalls in Gegenwart eines Verdünnungsmittels umsetzt,

oder

(j) 3-(4-Trifluormethyl-phenoxy)-benzylthioverbindungen der Formel

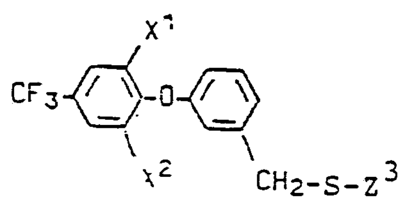

(XIa)

in welcher

$X^1$, $X^2$ und $Z^3$ die oben angegebene Bedeutung haben,

mit Oxidationsmitteln gegebenenfalls in Gegenwart eines Verdünnungsmittels umsetzt,

oder

(k) 3-(4-Trifluormethyl-phenoxy)-benzylhalogenide der Formel

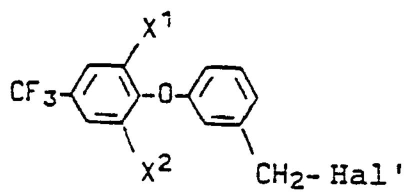

(XVI)

in welcher

$X^1$, $X^2$ und Hal' die oben angegebene Bedeutung haben,

α) mit Nucleophilen der Formel

$$M^1-Z \qquad (VII)$$

in welcher

$M^1$ und Z die oben angegebene Bedeutung haben,

gegebenenfalls in Gegenwart eines Säureakzeptors sowie gegebenenfalls in Gegenwart eines Verdünnungsmittels umsetzt,

oder

ß) mit Trialkylphosphiten der Formel

$$P(OR)_3 \qquad (VIII)$$

in welcher

R für Alkyl steht,

gegebenenfalls in Gegenwart eines Katalysators und gegebenenfalls in Gegenwart eines Verdünnungsmittels umsetzt und gegebenenfalls anschließend die dabei entstehenden Phosphonsäureester der Formel

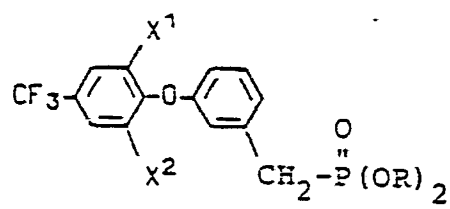

(XIb)

in welcher

$X^1$, $X^2$ und R die oben angegebene Bedeutung haben,

gegebenenfalls in Gegenwart eines Verdünnungsmittels verseift und gegebenenfalls die dabei entstehenden Säuren der Formel

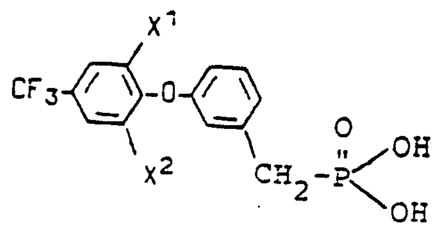

(XIc)

in welcher

$X^1$ und $X^2$ die oben angegebene Bedeutung haben,

mit einer Verbindung der Formel

$M^2X^3$ (IX)

in welcher

$M^2$ und $X^3$ die oben angegebene Bedeutung haben,

gegebenenfalls in Gegenwart eines Verdünnungsmittels umsetzt,

oder

(l) 3-(4-Trifluormethyl-phenoxy)-benzylamine der Formel

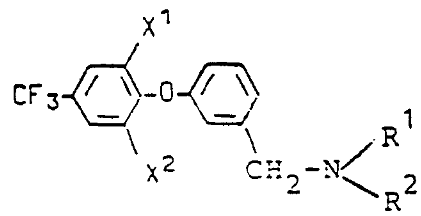

(XId)

in welcher

$X^1$, $X^2$, $R^1$ und $R^2$ die oben angegebene Bedeutung haben,

mit Halogeniden der Formel

$$Hal''-R^3 \quad (X)$$

in welcher

Hal" und $R^3$ die oben angegebene Bedeutung haben,

gegebenenfalls in Gegenwart eines Verdünnungsmittels umsetzt,

oder

(m) substituierte 3-(4-Trifluormethyl-phenoxy)-toluole der Formel

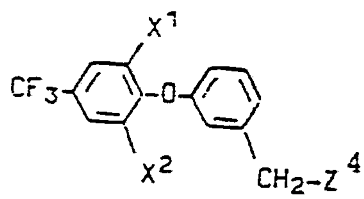

(XIe)

in welcher

$X^1$, $X^2$ und $Z^4$ die oben angegebene Bedeutung haben,

mit Alkalihydroxiden in Gegenwart eines Verdünnungsmittels umsetzt und die dabei entstehenden Carbonsäuresalze mit Mineralsäuren behandelt.